# EUROPEAN PATENT SPECIFICATION

(11) **EP 0 584 273 B1**
(45) Date of publication and mention of the grant of the patent: **30.12.1998**
(21) Application number: 92913635.6
(22) Date of filing: 04.05.1992
(51) Int. Cl.: A61K 39/40, C12P 21/02, C12P 21/08

(54) **ANTIBODY RECOGNIZING ENDOTHELIAL CELL LIGAND FOR LEUKOCYTE CR3**
Endothelzell-Ligand für den Leukocyten CR3 erkennender Antikörper
ANTICORPS RECONNAISSANT LE LIGAND DE CELLULES ENDOTHELIALES DU LEUCOCYTE CR3

(30) Priority: 03.05.1991 US 695613
(43) Date of publication of application: 02.03.1994
(73) Proprietor: THE ROCKEFELLER UNIVERSITY, New York New York 10021-6399 (US)
(72) Inventor: TUOMANEN, Elaine, New York, NY 10021 (US); Masure, H., Robert, New York, NY 10021 (US)
(74) Representative: Weinhold, Peter, Dr.
(86) International application number: US9203725
(87) International publication number: WO9219269

(56) References cited:
- PROC. NATL. ACAD SCI. vol. 86, April 1989, NATL. ACAD SCI., WASHINGTON, DC, US; pages 2637 - 2641; D.A. RELMAN ET AL.: 'Filamentous hemagglutinin of Bordetella pertussis: nucleotide sequence and crucial role in adherence' cited in the application
- CELL vol. 61, 29 June 1990, CELL PRESS, CAMBRIDGE, NA.; pages 1375 - 1382; D. RELMAN ET AL.: 'Recognition of a bacterial adhesin by an integrin: macrophage CR3 (alphaMbeta2, CD11b/CD18) binds filamentous hemagglutinin of Bordetella pertussis' cited in the application

## Description

### BACKGROUND OF THE INVENTION

Filamentous hemagglutinin (FHA) is a 220kD, non-fimbrial surface associated protein produced and secreted by Bordetella pertussis (BP). It is a necessary factor for BP to adhere to ciliated respiratory epithelial cells during whooping cough, Tuomanen et al, J. Infec. Dis. 152, 118-125 (1985). FHA has also been shown to interact with the integrin complement receptor 3 (CR3) on macrophages and other leukocytes, Relman et al, Cell 61, 1375-1382 (1990). CR3 is also known as Mac-1, α_{M}β₂ and CD 11b/CD18. The portions of FHA responsible for these two binding properties are distinct.

BP binds to two cell types during infection: leukocytes and ciliated cells. Adherence to cilia of the ciliated cells depends on recognition of carbohydrates such as galactose containing glycolipids. Tuomanen et al, J. Exp. Med. 168:267-277(1988).

The organism binds to leukocytes by two means. For the first, it binds to the integrin CR3, a step which precedes entry of the bacteria into the leukocyte, as discussed in more detail below. For the second, BP binds to leukocyte carbohydrates. BP also binds to cilia by recognizing a carbohydrate analogous to that on leukocytes. Galactose is the minimum requirement for a carbohydrate receptor. It is found, for example, in such blood group determinants as Lewis a.

There are two aspects of a BP infection. One is the invasion of the leukocytes which takes place when BP binds to the integrin of leukocytes. It is a protein/protein interaction. The other, adhesion of BP to the leukocyte or the cilia through a protein/carbohydrate interaction.

The FHA gene of BP has been sequenced, Relman et al, Proc. Natl. Acad. Sci. USA 86, 2637-2641 (1989) and Domenighini et al, Molec. Micro. 4,487-800 (1990), and a number of expression products have been produced, Delisse-Gathoye et al, Infect. Immun. 58, 2895-2905 (1990).

FHA and the integrin on leukocytes interact in a protein-protein recognition event. The interaction between FHA and leukocyte integrin involves recognition of the arginyl-glycyl-aspartyl sequence at positions 1097 to 1099 in FHA. This sequence will hereinafter be identified as the RGD sequence or simply, RGD, and the region of FHA or FHA segment on which it occurs as the RGD region. R, G and D are the standard one letter abbreviations for arginine, glycine and aspartic acid.

There are two natural molecules which bind to the integrin CR3 in a manner which appears to involve recognition of RGD. One molecule is the serum complement component C3bi, an opsonin. The other is the x-molecule(s) on endothelial cells.

It has long been known that leukocytes can invade or pass through vascular endothelial tissue by a process in which integrins, such as CR3, bind to receptors for integrins on the surface of endothelial cells as a step in a sequence of reactions which results in a widening of the junctions between such cells to permit passage by the leukocytes. The receptors for CR3 on endothelia are referred to herein as x-molecules. There may be one or more than one such x-molecule.

In addition to the ability to recognize C3bi and endothelial cells, CR3 also binds to Factor Ten of the coagulation cascade (Altieri et al, Science 254:1200-1202, 1992). The coagulation cascade is involved in inflammation since procoagulant activity arises on endothelial cells during infection or other noxious stimuli. Three regions of Factor Ten participate in recognition of CR3 and all three have homologous regions in FHA.

To summarize, FHA appears to represent a molecule with many binding regions for CR3, each region representing a natural analog in a eukaryotic protein.

### SUMMARY OF THE INVENTION

FHA or its regions can serve an anti-inflammatory function by acting as competitive inhibitors of CR3 recognition of endothelial cells (one region), C3bi (one region), or Factor Ten (four regions). Another separate and distinct region is the carbohydrate recognition domain (CRD) which allows the bacteria to adhere to cilia.

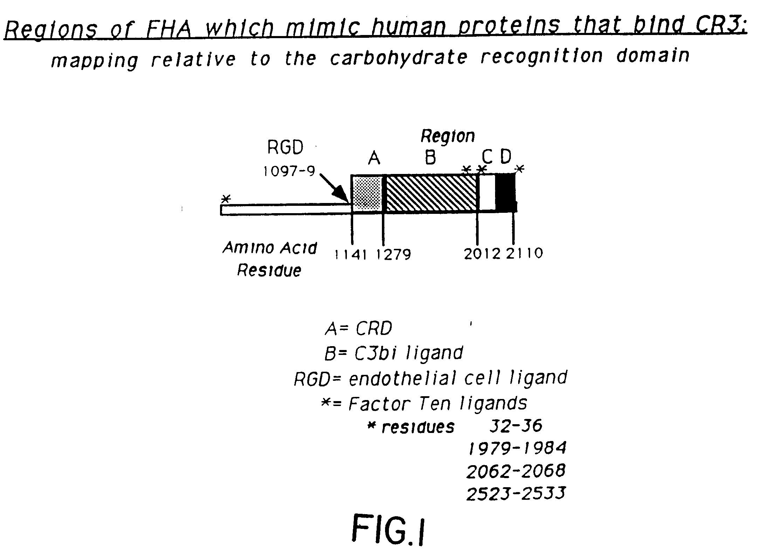

For convenience in discussing these various properties of FHA, each region is defined according to the scheme in Figure 1. Regions A, B, C and D are defined by antibodies as in Delisse-Gathoye et al, Infect Immun 58:2895-2905, 1990 and are bounded by the amino acid residues as shown. Region A contains the CRD. Region B contains the domain mimicking C3bi. Regions consistent with Factor Ten-like domains are represented by *. The region including the RGD (residues 1097-9) mimics the endothelial cell ligand for CR3 which participates in leukocyte transmigration.

It has been discovered that leukocytes use the same receptor, the integrin CR3, to bind to FHA, C3bi, Factor Ten and the x-molecule of endothelial cells. This establishes that FHA and these molecules are functionally related. They are also antigenically related and therefore may be structurally related in that FHA contains the amino acid triplet RGD and the x molecule may contain the RGD triplet or a segment which acts like the RGD triplet. As a result, some antibodies to FHA may cross react with endothelial cells. Similarly, FHA contains 4 regions with sequence similarity to Factor Ten. The specific leukocyte integrin involved in the binding is believed to be CR3, otherwise known as Mac-1, CD11b/CD18. Moreover, there is species cross reactivity. Thus antibodies to FHA raised in a goat, mouse, guinea pig or human will react with and bind to C3bi, Factor Ten or endothelial cells of rats, rabbits and humans. There may be one or several distinct molecules on endothelial cells which function as x-molecules by binding anti-FHA antibodies.

Significant consequences of this important discovery are:
1. Peptides which contain or mimic the RGD region, a Factor Ten region or the C3bi region of FHA will bind to the integrin of leukocytes, thereby preventing adherence of the leukocyte to the endothelial cells as a procedure for inhibiting the inflammatory process.
2. Antibodies to FHA will bind to the homologous eukaryotic proteins disturb their function. In the case of the C3bi-like region, antibodies will bind C3bi or other related complement components and render them less effective for opsonization. In the case of the Factor Ten like regions, the antibodies will disturb coagulation and prevent amplification of inflammation by the coagulation cascade. In the case of the RGD region, antibodies to FHA will bind to endothelial cells of, for example, the blood brain barrier and selectively open the junction between the cells in a manner analogous to the opening of the endothelia during leukocyte diapedesis thereby making possible the entry of desirable therapeutic agents into the cerebrospinal fluid of the subarachnoid space and into the brain parenchymsa without simultaneously admitting the entrance of leucocytes.

The present invention takes advantage of this discovery by utilization of FHA or regions of FHA or antibodies thereto to prevent CR3 functions in inflammation as is exemplified by procedures 1 and 2 below.

This invention is directed to peptides derived from FHA, analogs of such peptides and antibodies to such peptides all of which are capable of inhibiting binding between CR3 and its natural ligands. It is also directed to pharmaceutical compositions containing the products and to therapeutic use of such products to inhibit or prevent such binding and to other uses which flow from these basic properties.

The understanding of this invention will be facilitated if certain of the terms used in the description thereof are defined.

The term "peptide" is used in the most generic sense to include those peptides which contain only a few amino acid residues, e.g. 5 or more, polypeptides containing 20 or more amino acids and proteins such as mutants and segments of FHA. The term encompasses mutants of FHA and segments thereof including addition, deletion and substitution mutations. The term is, perhaps, best understood from the function the peptides perform as discussed herein. These functions include inhibition of binding between the CR3 integrin and its natural ligands, Factor Ten, C3bi, or an x-molecule on endothelial cells. They may do so because they contain an RGD triplet or a moiety which mimics an RGD triplet. They may also function by mimicking C3bi or Factor Ten peptides. These peptides are useful in control of inflammation.

The term "antibodies" encompasses both polyclonal and mononclonal antibodies which bind to C3bi (or related complement components) Factor Ten or x-molecules on brain endothelium. The preferred antibody is a monoclonal antibody. The term antibody is also intended to encompass mixtures of more than one antibody (e.g., a cocktail of different types of monoclonal antibodies). The term antibody is further intended to encompass whole antibodies, single chain antibodies, chimeric antibodies comprising portions from ore than one species, chimeric proteins comprising a functional portion of antibody coupled by covalent or recombinant techniques to an intact protein or functional portion thereof that is not of antibody origin (i.e. a chimeric antibody-protein), bifunctional antibodies, biologically functional fragments of the aforementioned, etc. Biologically functional antibody fragments which can be used are those fragments sufficient for binding of the antibody fragment to FHA or the x-molecule as well as Factor Ten and C3bi.

The chimeric antibodies can comprise portions derived from two different species (e.g., human constant region and murine binding region). The portions derived from two different species can be joined together chemically by conventional techniques or can be prepared as a single contiguous protein using genetic engineering techniques. DNA encoding the proteins of both portions of the chimeric antibody can be expressed as a contiguous protein.

The term "RGD region" includes any RGD containing segment of a molecule whether that molecule is one of high molecular weight such as FHA, or the x-molecule, or a peptide of relatively low molecular weight. There are molecules which mimic the RGD region and these may be involved in the practice of this invention. Native molecules or chemically treated molecules or derivatives are included in this definition.

The term "block the RGD region" will be used to describe peptides which inhibit adhesion between leukocytes and endothelial cells for any of a number of reasons. They may do so because they bind to all three of the amino acid residues of RGD or to only one or two of them. They may also do so because they bind to molecular segment(s) neighboring the RGD region in such a manner that one or all of the RGD residues is prevented from reacting with an integrin.

The term "Factor Ten like region" refers to any segment of a molecule containing sequences GYDTKQEDG, IDRSMKTRG or GLYQAKRFKVG or highly homologous sequences.

The antibodies useful in the practice of this invention can be raised against whole bacteria containing FHA or derivatives or against native FHA or chemically treated FHA or derivatives, and this will be the procedure usually employed to produce polyclonal antibodies. They can also be raised to mutants of FHA or fragments or segments of mutants of FHA thereof produced either by genetic manipulation of the FHA gene or by chemical or enzymatic cleavage of the whole protein or a fragment of the protein. They can be produced, and this is the preferred method, by raising monoclonal antibodies to the expression products from segments of the FHA gene or mutants of such segments according to the method of Delisse-Gathoye et al, supra. The expression products are proteins or peptides containing a sufficient number of amino acid residues to elicit an antibody response alone or when attached to other antigenic determinants. The antibodies can be produced in accordance with well known procedures for the production of monoclonal antibodies. These monoclonal antibodies are the preferred therapeutic agents for the practice of this invention.

The presently preferred monoclonal antibodies are 12.1 F9, 13.6E2, 12.6F8 and 12.2 B11 produced by the methods described by Delisse-Gathoye et al., supra. A preferred polyclonal antibody is goat antiserum to FHA produced by the Cowell procedure as described in Tuomanen et al. cited above.

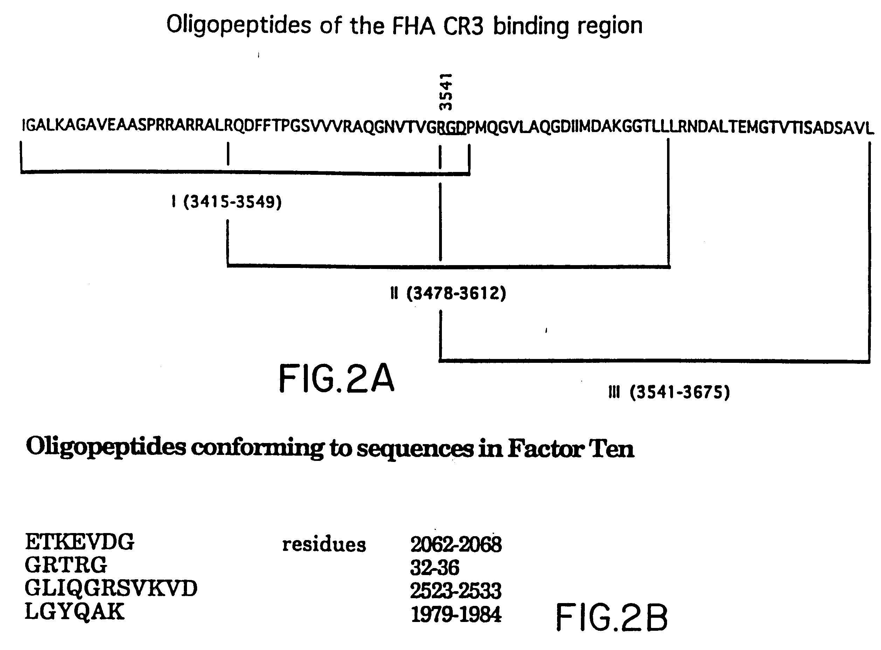

The presently preferred antiinflammatory products of this invention are peptides containing from about five to about forty-five amino acid residues, preferably about twelve to thirty five and their analogs, especially those shown in Figs. 2A and 2B. These are preferred because they are relatively small molecules which can be readily prepared in pure form by chemical synthesis. It will be apparent as the description of the invention proceeds that the invention is not limited to these peptides, but these presently appear to be preferred.

A peptide of the invention will be useful if it is capable of reducing or inhibiting adhesion between leukocytes and endothelia or of raising antibodies having these properties.

It will be clear from this definition that peptides of this invention may contain amino acid derivatives comprising single or multiple amino acid additions, deletions and/or substitutions compared to the amino acid sequence of FHA. The peptides contemplated herein may be chemically synthesized such as by solid phase peptide synthesis or may be prepared by subjecting the designated polypeptide to hydrolysis or other chemically disruptive processes whereby fragments of the molecule are produced. Alternatively, the peptides could be made in vitro or in vivo using recombinant DNA technology. In this case, the peptides may need to be synthesized in combination with other proteins and then subsequently isolated by chemical cleavage or the peptides may be synthesized in multiple repeat units. Furthermore, multiple antigen peptides could also be prepared according to the method of Tam et al, J. Am. Chem. Soc. 105 6442-6445 (1988). The selection of a method of producing the subject peptides will depend on factors such as the required type, quantity and purity of the peptides as well as ease of production and convenience.

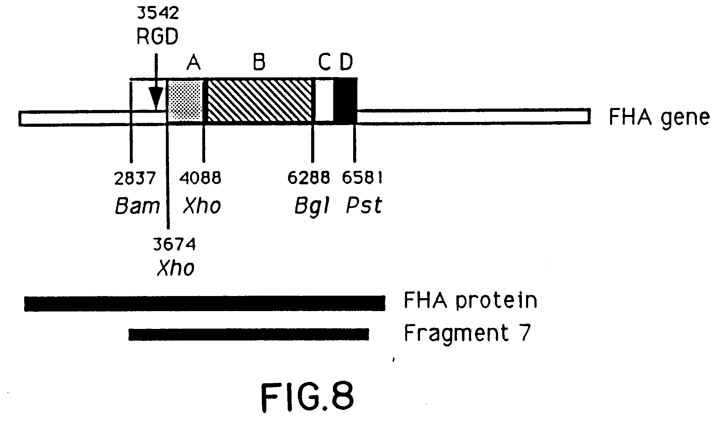

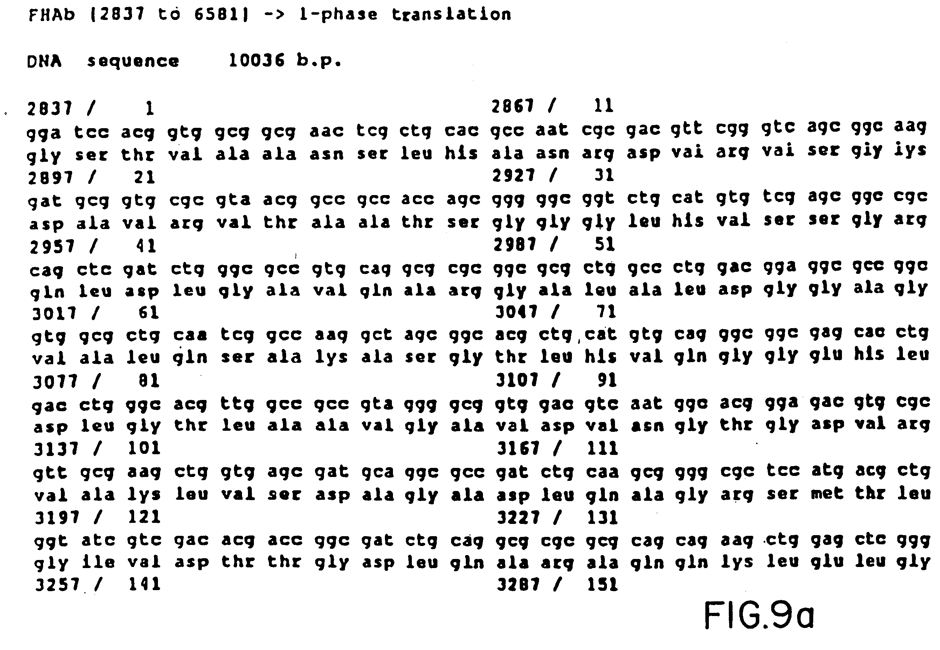

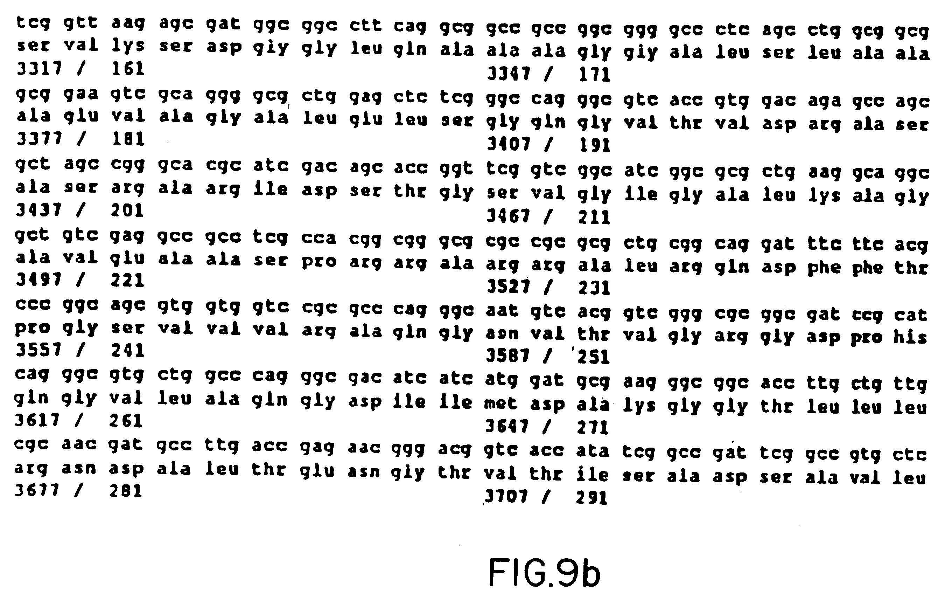

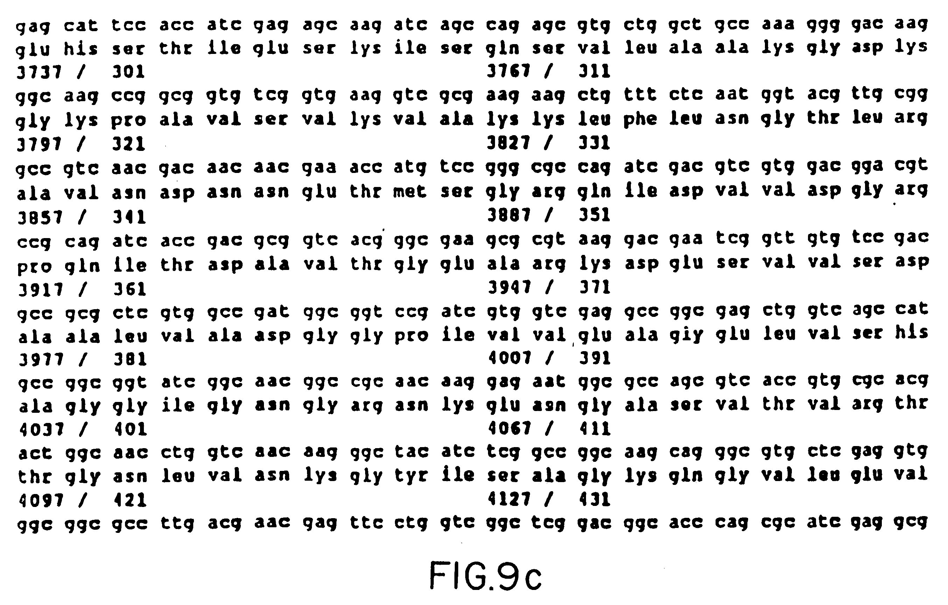

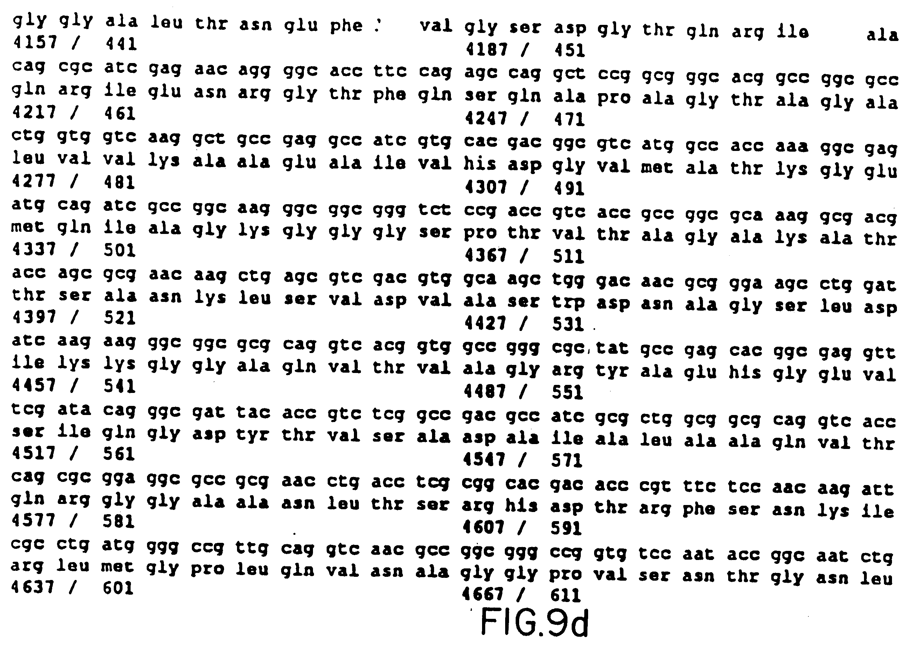

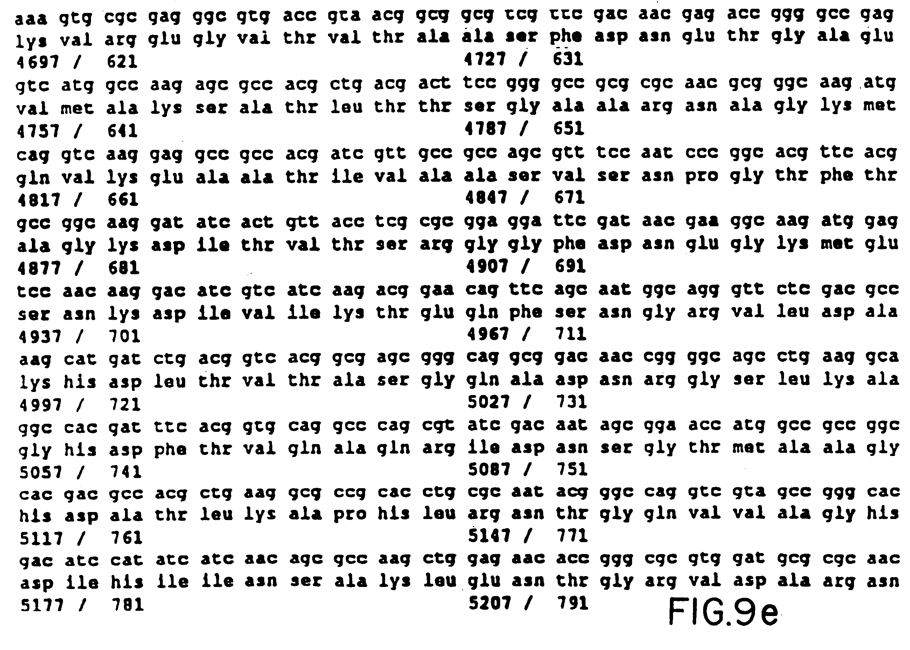

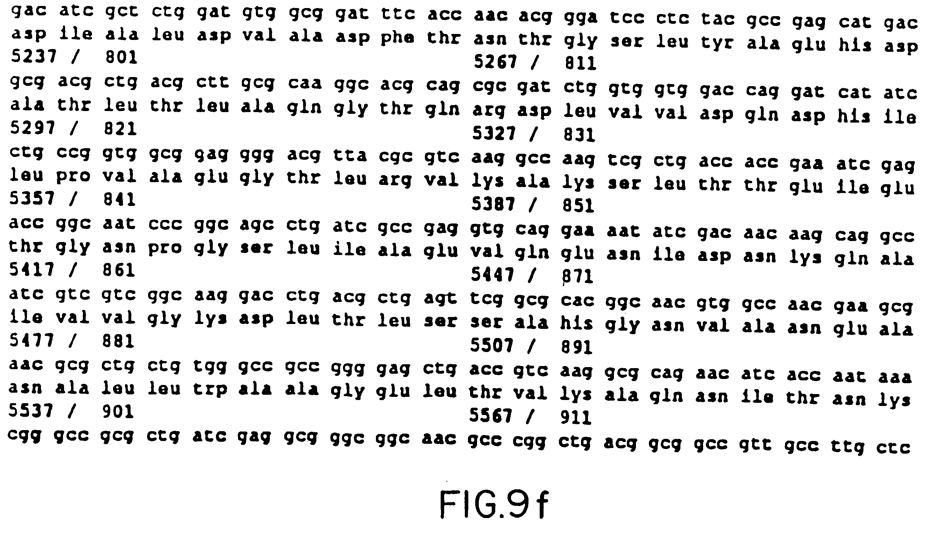

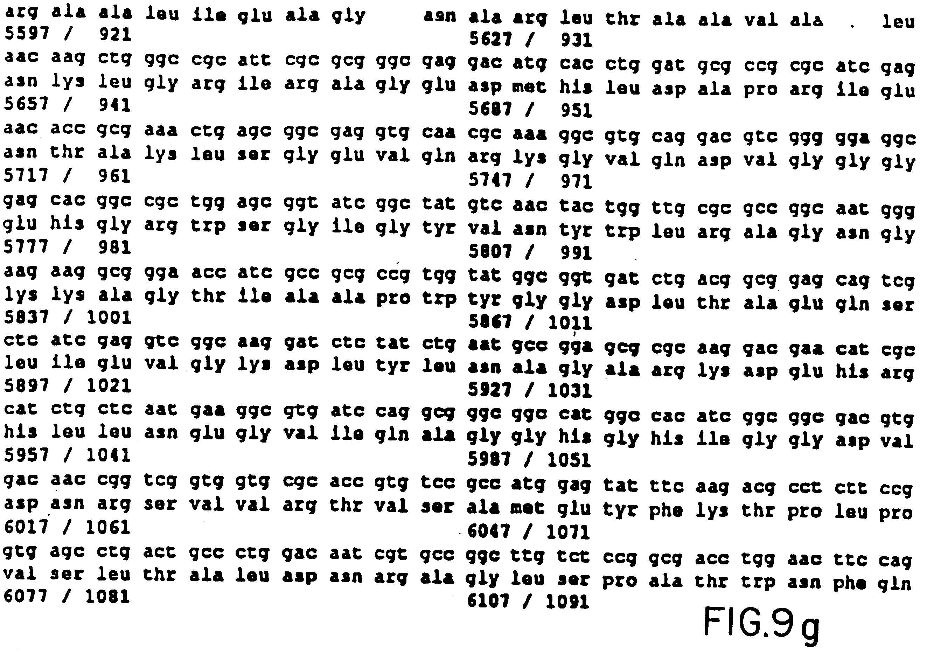

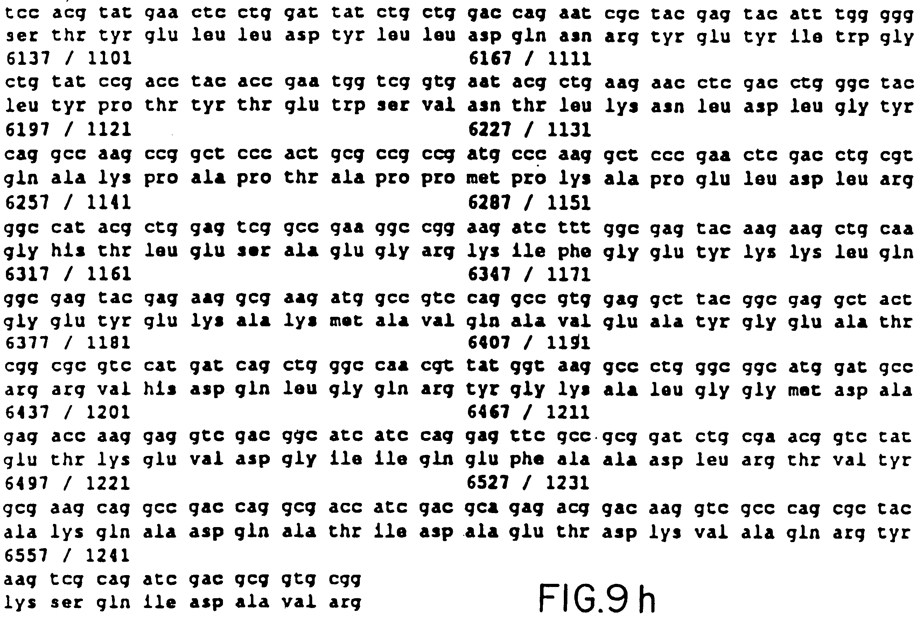

Delisse-Gathoye et al., supra have described a number of expression products of the FHA gene. One of them, Fragment 7 is defined by the Bam HI site at position 2837 and the Pst 1 site at position 6581 as shown in Fig. 8 which also shows other segments of the fraction defined by other restriction enzymes. This region (Fig. 9) expresses an FHA segment which contains the RGD region at the positions corresponding to positions 1097 to 1099 of FHA.

The use of these peptides in vivo may first require their chemical modification since the peptides themselves may not have a sufficiently long serum and/or tissue half-life. Such chemically modified peptides are referred to herein as "analogs". The term "analog" extends to any functional chemical equivalent of a peptide characterized by its increased stability and/or efficacy in vivo or in vitro in respect of the practice of the invention. The term "analog" is also used herein to extend to any amino acid derivative of the peptides as described herein.

Analogs of the peptides contemplated herein include, but are not limited to, modifications to side chains, incorporation of unnatural amino acids and/or their derivatives during peptide synthesis and the use of crosslinkers and other methods which impose conformational constraints, on the peptides or their analogs.

Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6, trinitrobenzene sulfonic acid (TNBS); acylation of amino groups with succinic anhydride and tetrahydrophthalic anhydride; and pyridoxylation of lysine with pyridoxal-5'-phosphate followed by reduction with NaBH₄.

The guanidino group of arginine residues may be modified by the formation of heterocyclic condensation products with reagents such as 2,3-butanedione, phenylglyoxal and glyoxal.

The carboxyl group may be modified by carbodiimide activation via O=acylisourea formation followed by subsequent derivatisation, for example, to a corresponding amide.

Sulfydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulfonic acid, phenylmercury chloride, 2-chloromercuric-4-nitrophenol and other mercurials; and carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids.

Crosslinkers can be used, for example, to stabilize 3D conformations, using homo-bifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH). In addition, peptides could be conformationally constrained by, for example, incorporation of C and N α-methylamino acids, introduction of double bonds between C and C atoms of amino acids and the formation of cyclic peptides or analogs by introducing covalent bonds such as forming an amide bond between the N and C termini, between two side chains or between a side chain and the N or C terminus.

The peptides of the invention or their analogs may be single length or in tandem or multiple repeats. A single type of peptide or analog may form the repeats or the repeats may be composed of different molecules including suitable carrier molecules.

The present invention, therefore, extends to peptides or polypeptides and amino acid and/or chemical analogs thereof corresponding to regions of the RGD domain.

### BRIEF DESCRIPTION OF THE FIGURES

Fig. 1 shows regions of FHA which mimic human proteins that bind to CR3 with the mapping relative to the CRD.
Figs. 2A and 2B are representative of peptides which will block leukocyte adherence to endothelia or interfere with coagulation.
Figs. 3-5 illustrate the use of FHA, peptides or antibodies of the invention as antiinflammatory agents.
Figs. 6 and 7 illustrate the use of antibodies to FHA to enhance vascular permeability.
Fig. 8 is a representation of Fragment 7 of FHA as defined by Delisse-Gathoye et al, supra.
Fig. 9 shows the sequence for the gene encoding Fragment 7 and for Fragment 7 itself.
Fig. 10 shows the relative ability of anti-FHA antibodies to bind to C3bi.
Fig. 11 and 12 refer to Examples 5 and 6, respectively.

### DETAILED DESCRIPTION OF THE INVENTION

The utilities which may be realized from the recognition that FHA, C3bi, Factor Ten and the x-molecule are structurally and functionally related and the recognition of the important consequences mentioned above will now be discussed.

### 1. FHA and peptides which contain or mimic the RGD region of FHA or the Factor Ten region of FHA will bind to integrins of leukocytes to inhibit the inflammatory process.

Leukocytes, such as monocytes, and polymorphonuclear leukocytes (PMN), circulate in the blood and normally do not adhere to the endothelium. Upon the introduction into the tissue of (1) an infectious agent, (2) fragments that result from the death of an infectious agent, or (3) another inflammatory substance, leukocytes, such as PMN, are induced to bind to the endothelium and then migrate into the tissues. This is a two step process in which the leukocyte initially binds to receptors on the endothelium, including the x-molecule. One effect of the binding is that the cell junctions in the endothelium open. This permits the leukocyte, in the second step, to move from the x-molecule, through the junctions and into the tissue. Since PMN can recognize and kill many infectious agents, the passage of leukocytes through the endothelium and into the tissue is a protective mechanism. However, in many disease circumstances, leukocytes react in an exaggerated and deleterious fashion. They may bind so avidly to endothelium as to occlude blood flow. Once in the tissues, they secrete proteases, reactive oxygen intermediates, and other toxic molecules which not only kill infectious agents, but also can result in extensive tissue damage. In addition, they trigger release of inflammatory mediators that alter vascular tone and permeability, and that recruit additional leukocytes to the site, thus perpetuating inflammation.

As stated above, FHA binds to CR3 leukocytes through three domains, namely RGD, Factor Ten like regions and C3bi like regions for which the integrin on the leukocyte surface acts as a receptor. When it does so, it prevents the leukocytes from using the integrins to adhere to the endothelium and initiating an inflammatory process.

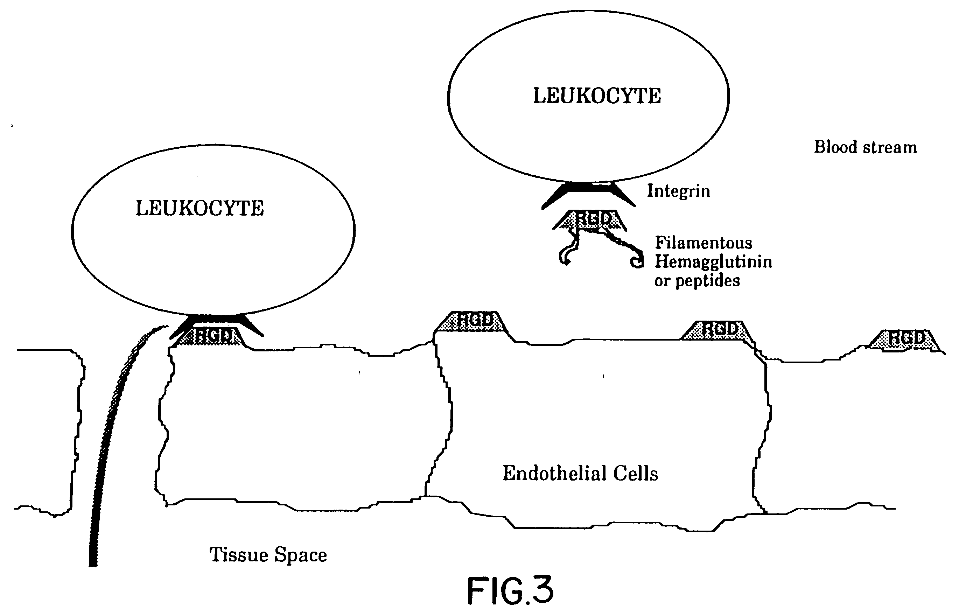

This process is schematically illustrated for the x-molecule in Fig. 3. The figure shows a portion of the endothelium constructed from adjacent endothelial cells with apposed cell junctions. The sketch shows leukocytes in the blood stream together with FHA or segments thereof.

The left side of the Figure illustrates the normal adhesive reaction between an integrin such as CR3 on a leukocyte and the RGD or RGD mimicking region of the x-molecule of the endothelium. The result of the reaction, as shown by the arrow, is that a cell junction opens and the leukocyte moves from the RGD into the tissue.

The right side of the sketch shows that the presence of FHA or peptides acting like FHA in the blood stream prevents this reaction because they react with the integrin and prevent it from binding to the x-molecule. This has the effect of preventing the interaction of the x-molecule and the leukocyte integrin so that the leukocyte cannot pass through the endothelium into the tissue.

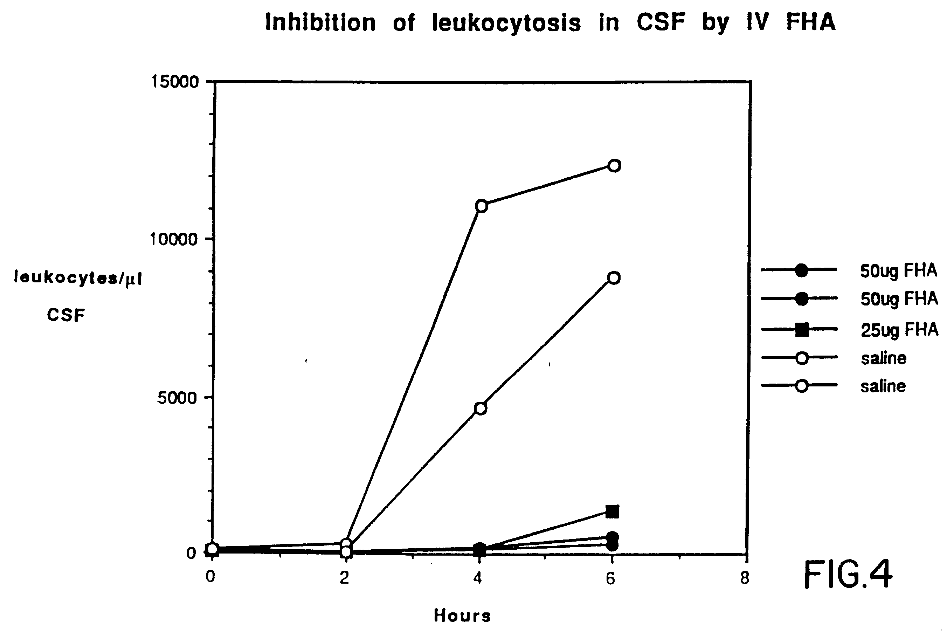

The agent which will achieve this desirable result may be native FHA or a segment thereof. Typically, it will be a relatively low molecular weight peptide containing one of two motifs; 1) an RGD region or an RGD mimicking region or 2) a Factor Ten like region. It may contain, for example from about 5 to 20 amino acid residues or even more. One example of such a peptide is described by Relman et al in the Cell article cited above. It is Thr-Val-Gly-Arg-Gly-Asp-Pro-His-Gln. Other examples are shown in Fig. 2. Fig. 4 illustrates that intravenous administration of FHA in an experimental model of meningitis decreases inflammation as evidenced by lower numbers of leukocytes in cerebrospinal fluid.

An infection in which leukocyte mediated damage contributes to morbidity and mortality of disease is bacterial meningitis. Depending upon the infecting organism, thirty percent of the cases of meningitis per year die despite sterilization of the infection by antibiotics. Over fifty percent of the survivors have permanent and severe sequelae such as paralysis, deafness, and learning disabilities. Obviously, the prevention and/or diminishment of such damage would greatly enhance the quality of life for the survivors of this disease.

Activated leukocytes also contribute to cerebral edema and blood-brain barrier injury. Neutropenic animals (animals in which the leukocytes have been artificially diminished) have been found to have improved survival rates in experimentally induced disease. A high amount of inflammation in the subarachnoid space correlates directly with a poor outcome of disease. Inhibition of the accumulation of leukocytes in cerebrospinal fluid directly correlates with improved morbidity and mortality of experimental pneumococcal meningitis and of Haemophilus influenzae meningitis and bacteremia in children.

Clearly, an agent which would inhibit the influx of leukocytes into infected sites would be a therapeutic tool of immense value particularly if non-leukocyte mediated defense systems are left functionally intact. It would further be beneficial to block leukocyte diapedesis only at inflammed sites and not at other sites throughout the body. Thus treatment directed at inflammed endothelia would be advantageous over that directed to leukocytes.

The use of antibiotics magnifies the deleterious effects of inflammation during infectious diseases. This is due to the mechanism by which such agents exert their antiinfective effects. For example, following the administration of a beta-lactam antibiotic (or another cell-wall directed antibiotic), the bacteria disintegrate due to lysis by the antiinfective agents. The resulting fragments of bacteria initiate a dramatically enhanced inflammatory response. Earlier research has indicated that inhibition of this enhanced level of inflammation correlates with improved morbidity and mortality, Tuomanen et al., J. Infect. Dis., 155, 985-990 (1985) and Kadurugamuwa, Program and Abstracts of the 27th ICAA Meeting, p. 205 (1987). In pneumococcal meningitis, for instance, mortality can be directly correlated with the amount of meningeal inflammation, McAllister et al., J. Infect. Dis., 132, 355-360 (1975). Thus, a method of dampening inflammation during the course of therapy with an antibiotic would be advantageous in treating infections, particularly meningitis, septic arthritis, and endophthalmitis.

The process of this invention will be useful in treating inflammation caused by any of a variety of infective agents, including gram-positive and gram-negative bacteria as well as viruses and fungi. Particularly targeted infections are those which are susceptible to treatment with beta-lactam antibiotics, or antiviral agents such as Haemophilus influenzae B; N. meningitidis b; pneumococci, e.g., Streptococcus pneumoniae; Escherichia coli; Staphylococcus epidermidus; Staphylococcus aureus; group B Streptococci; Salmonella; Bacillus subtilis; Pseudomonas aeruginosa; and Herpes virus.

The infected tissue which is the target of the present invention can likewise be tissue in any body site susceptible to inflammation caused by the above-described infective agents. The method of the present invention is, however, particularly adaptable to the treatment of infected tissue of the central nervous system, lung, kidney, joints, endocardium, eyes and ears, with the treatment of the cerebrospinal fluid and articular fluid being highly preferred embodiments.

One particularly susceptible tissue for which the present invention is uniquely suited is the tissue of the central nervous system. The vascular endothelium in the brain is morphologically different from that in other tissues in that endothelial cells are joined by tight junctions thereby creating a blood-brain barrier which prevents molecules the size of proteins from passing from blood into the cerebrospinal fluid. They are particularly useful in the treatment of meningitis infections, including those arising from pneumococci, Haemophilus influenzae B, N. meningitidis b and Escherichia coli, group B Streptococcus and Staphylococci.

Additionally, the ingress of leukocytes into articular fluid can be prevented by administration of a therapeutic amount of a selected peptide of the present invention. In cases where the inflammation of an infection migrates to the joints, e.g. arthritis, this method can be utilized to alleviate the inflammation by preventing the ingress of leukocytes into the articular fluid.

The process of this invention is useful in the control of inflammation arising from substantially any source including, for example autoimmune disease.

A further method of the present invention is that of reducing or eliminating inflammation in an infectious disease caused by the administration of an antiinfective agent for that disease which comprises the simultaneous administration of an effective amount of antiinfective agent and an effective amount of peptide or an active fragment thereof to a patient in need of such therapy. Due to the mechanism of their therapeutic activity, antiinfective agents, and particularly beta-lactam antibiotics, cause additional inflammation as a result of their therapeutic effect. Although such antiinfective agents sterilize a given infection, they cause release of the cell wall and/or endotoxin of the infecting agent. Such bacterial components initiate an inflammatory response in the infected tissue. It is this inflammation which contributes significantly to the tissue damage that is the long-term consequence of most infections.

The term "simultaneous administration" as used herein means that therapeutic amounts of the antiinfective agent and the peptide are administered within a time period where they influence each other. Thus the anti-infective agent may be administered at the same time or before or after the peptide.

Reduction or elimination of inflammation in infectious diseases results in a diminution of the neurological damage that usually accompanies such infections. Since the peptides of this invention possess the unique ability to block movement of leukocytes across the blood-brain barrier, they are uniquely suited to treat infections where the causative infective agent is Haemophilus influenza B, N. meningitidis b, or a pneumococci such as Streptococcus pneumoniae. Such infections are generally treated with an aminoglycoside such as gentamicin or a beta-lactam antibiotic such as a penicillin or cephalosporin.

Due to the ability of FHA to reduce or eliminate inflammation in an infectious disease caused by the administration of an anti-infective agent, FHA or active fragment thereof can be combined in a single unit dosage form with the anti-infective agent for convenience of administration. Such dosage form is most preferably an intravenous dosage form since most anti-infective agents, particularly the beta-lactam antibiotics, are available in a suitable chemical form for administration via the intravenous route. This is also the preferred route of administration for FHA or its peptides of the invention. Typically, the anti-infective agent and one or more FHA peptides can be combined in a single ampoule solution. Where this is not possible, the anti-infective agent and the peptide can be packaged separately and mixed just prior to injection. Administration can likewise be via a mixture with any standard intravenous solution, i.e., normal saline.

The amount of anti-infective agent in the dosage form is dependent upon the particular anti-infective agent being utilized and the particular infection being treated. The amount of the peptide utilized in dosage form can range from about 1 to about 1,000 mg, with 10-100 mg per dosage unit being highly preferred. Dosages can be administered one to four times daily, with continued therapy for as long as the infection persists.

This process is also applicable to targeting therapeutic agents to leukocytes. The desired therapeutic agent, for example an anti-leukemic agent, an immunomodulator or other known drug may be bonded to a selected peptide by any selected process, and it will be carried to the leukocyte by the peptide because the peptide binds to CR3. CR3 is restricted to leukocytes and thus limits distribution of peptide linked agents to leukocytes. Furthermore, ligands bound to CR3 initiate ingestion of the ligand so the peptide linked agent could be delivered to and then taken up by the leukocyte.

### 2. Antibodies to FHA may by employed to block inflammation or induce blood brain barrier permeability.

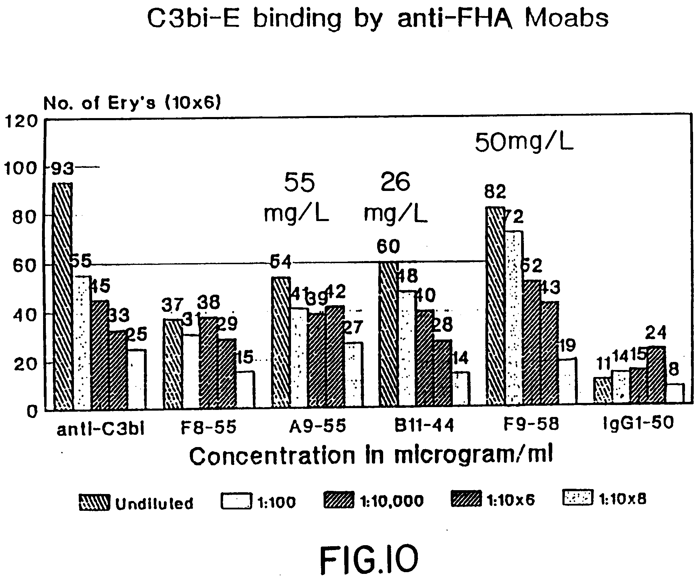

Since FHA contains domains that resemble C3bi, Factor Ten and the x molecules of endothelial cells, antibodies to FHA bind to these natural molecules and can be used to disturb their function. The data and description for antibodies binding to C3bi is reported in Example 8 and Fig. 10. This section will illustrate two phenomena:
2a) Antibodies to Factor Ten domains block leukocyte recruitment, the preferred antibody being 12.1B11.
2b) Antibodies to the RGD region bind to endothelial cells and enhance permeability of the blood brain barrier, the preferred antibody being 13.6E2.

### 2a). Antibodies to FHA which block leukocyte recruitment, i.e, 12.1B11.

During an inflammatory process the coagulation cascade is activated by the expression of coagulation factors on the surface of an endothelium. This leads to a net pro-coagulant state at the endothelial surface and promotes the deposition of fibrin and clotting. These processes result in the localized thrombotic events characteristic of advanced inflammation and contribute to tissue damage by occluding blood flow leading to tissue anoxia. Factor Ten is a coagulation cascade component and it interacts with CR3 on leukocytes to promote the association of leukocytes with cells which harbor procoagulant proteins on their surface, such as an endothelium. Factor Ten has three regions which bind to CR3 as shown by the ability of three peptides to competitively inhibit the binding of CR3 bearing tissue culture cells to purified Factor Ten. These three peptides are GYDTKQEDG (366-373), IDRSMKTRG (422 to 430) and GLYQAKRFKVG (238-246) as described by Altieri et al, Science, supra.

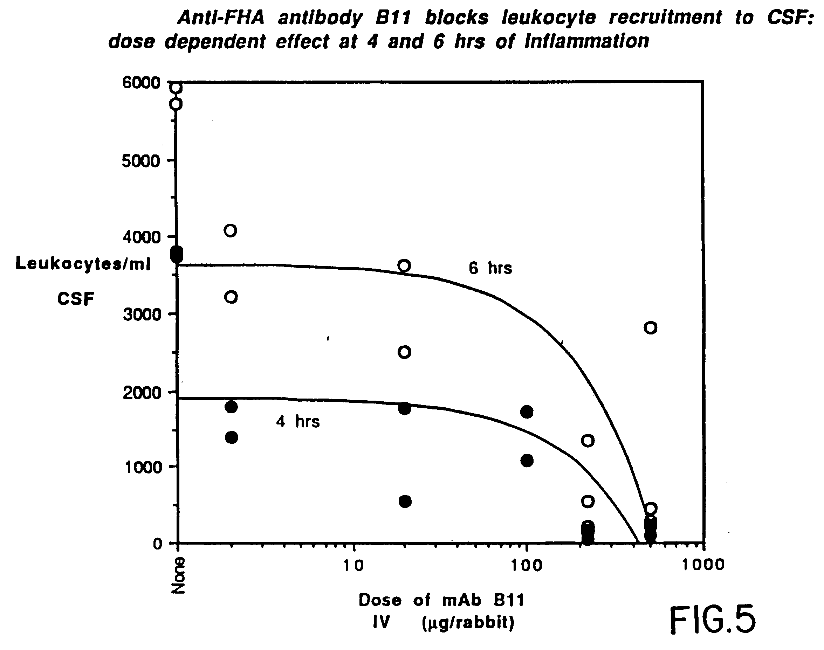

FHA contains four regions of significant sequence similarity to these regions of Factor Ten. They are shown in Figure 2B. Based on this sequence similarity, FHA has antiinflammatory activity as documented in Fig. 4. This similarity makes it clear that antibodies to FHA which bind to these regions would disturb leukocyte recruitment. This is to be distinguished from anti-FHA antibodies that bind to endothelia by recognizing an x molecule which is an endothelial cell component (see below). In the case of anti-FHA antibodies which bind to the Factor Ten like regions, the antibodies do not bind directly to endothelial cell components but rather to the coagulation components captured on the endothelial cells during inflammation. This is illustrated by the activity of anti-FHA antibody 12.1B11 which does not bind to capillaries but is highly antiinflammatory when administered intravenously into rabbits with meningitis. This is shown in Figure 5. Various doses of antibody were given to rabbits and the number of leukocytes recruited into the cerebrospinal fluid in response to pneumonoccal meningitis was determined. Those animals receiving even low doses of antibody 12.1B11 showed inhibition of leukocyte accumulation in cerebrospinal fluid.

### 2b). Antibodies to the RGD region bind to endothelial cells and enhance blood brain barrier permeability

The antibodies of this invention bind to and block the RGD or RGD mimicking region of endothelial cells including those of the blood brain barrier (BBB) and facilitate the passage of water soluble molecules including, for example, therapeutic agents through the BBB and into the cerebrospinal fluid (CSF).

Anti-FHA antibodies apparently bind to molecules on capillary endothelial cells known as endothelial cell ligands (to distinguish them from the FHA receptors found on leukocytes). These EC ligands have determinants that are exposed on the vascular surface of endothelial cells. This feature is inferred from the ability of intravenously administered anti-FHA antibodies to bind to vessels as measured immunohistochemically. These EC ligand determinants do not require stimulation by cytokines in order to be expressed or incorporated on the vascular surface of capillary endothelial cells. Antibodies to ICAM-1 do not appear to bind to these EC ligands. This indicates that the EC ligands are not ICAM-1. Immune blot analysis of the proteins from purified human cerebral capillaries with anti-FHA antibodies indicates that these antibodies bind to two novel polypeptides of apparent molecular size of 64 and 52 kilodaltons.

When the anti-FHA antibody binds to EC ligands, an apparently transient opening of the BBB to therapeutic agents results. That is, in response to intravenous administration of these anti-FHA antibodies, the penetration of therapeutic agents into the brain is enhanced in a time dependent and reversible manner. In addition, binding of intravenously administered anti-FHA antibodies to EC ligands results in an inhibition of leukocyte diapedesis into the brain even though the BBB permeability to therapeutic agents is increased as a result of such binding. These features of anti-FHA antibodies will now be illustrated.

It is a particular advantage of the invention that certain antibodies within its scope permit such passage without concurrent penetration of leukocytes into brain or cerebrospinal fluid (CSF).

Examples 5 and 6 illustrate the ability of the antibodies of this invention to assist in the penetration of the blood brain barrier by penicillin.

The BBB is a continuous boundary between the blood and both the interstitial fluid (IF) and the CSF of the brain. It is composed of a layer of endothelial cells, the cerebral capillary endothelium, that serves as an effective barrier against the entry into the brain's tissue of serum components of both high and low molecular sizes. The restriction against entry of such substances into the brain and the CSF is due to the structure of the cerebral capillary endothelium in which the anatomically tight junction seals spaces between adjacent endothelial cells.

In a normal (healthy) state, the only substances capable of traversing the BBB to enter the CSF tend to be relatively hydrophobic (lipid-like). Substances which are hydrophylic (water-soluble) penetrate the BBB much less effectively or not at all. Such water-soluble and poorly penetrating substances encompass a whole range of molecules extending from molecules as large as albumin to ions as small as sodium.This poor permeability of BBB by many potentially useful drugs poses a severe limitation on the treatment of diseases of the brain tissue and CSF. It is therefore of paramount clinical significance to develop products and methods which would "open" the BBB and allow access to the brain tissues and CSF by agents which are known to be effective in treating or diagnosing brain disorders but which, on their own, would not be able to traverse the BBB. Certain antibodies of this invention will achieve that end.

This invention provides a method for increasing the permeability of the blood-brain barrier of a host to a molecule present in the host's bloodstream. The host can be any animal which possesses a central nervous system (i.e., a brain). Examples of hosts include mammals, such as humans and domestic animals (e.g., dog, cat, cow or horse), as well as animals intended for experimental purposes (e.g., mice, rats, rabbits).

The molecule in the host's bloodstream can be exogenous to the host. For example, it can be a neuropharmaceutical agent which has a therapeutic or prophylactic effect on a neurological disorder. Examples of neurological disorders include cancer (e.g., brain tumors), Acquired Immunodeficiency Syndrome (AIDS), epilepsy, Parkinson's disease, multiple sclerosis, neurodegenerative disease, trauma, depression, Alzheimer's disease, migraine, pain, or a seizure disorder.

Classes of nueropharmaceutical agents which can be used in this invention include antibiotics, adrenergic agents, anticonvulsants, nucleotide analogs, chemotherapeutic agents, anti-trauma agents and other classes of agents used to treat or prevent a neurological disorder. Examples of antibiotics include amphotericin B, gentamycin sulfate, pyrimethamine and penicillin. Examples of adrenergic agents (including blockers) include dopamine and atenolol. Examples of chemotherapeutic agents include adriamycin, methotrexate, cyclophosphamide, etoposide, carboplatin and cisplatin. An example of an anticonvulsant which can be used is valproate and an anti-trauma agent which can be used is superoxide dismutase. Nucleotide analogs which can be used include azido thymidine (AZT), dideoxy Inosine (ddI) and dideoxy cytodine (ddc).

The molecule in the host's bloodstream can also be diagnostic imaging or contrast agents. Examples of diagnostic agents include substances that are labelled with radioactivity, such as 99-Tc glucoheptonate.

The administration of exogenous molecules and/or antibody to FHA to the host's bloodstream can be achieved parenterally by subcutaneous, intravenous or intramuscular injection or by absorption through a bodily tissue, such as the digestive tract, the respiratory system or the skin. The form in which the molecule is administered (e.g., capsule, tablet, solution, emulsion) will depend, at least in part, on the route by which it is administered.

The administration of the exogenous molecule to the host's bloodstream and the administration of antibody to FHA can occur simultaneously or sequentially in time. For example, a therapeutic drug can be administered orally in tablet form while the intravenous administration of the antibody is given 30 minutes later. This is to allow time for the drug to be absorbed in the gastrointestinal tract and taken up by the bloodstream before the antibody is given to increase the permeability of the blood-brain barrier to the drug. On the other hand, the antibody can be administered before or at the same time as an intravenous injection of the drug. Thus, the term "co-administration" is used herein to mean that the blood-brain barrier permeabilizing antibody and the exogenous molecule will be administered at times that will achieve significant concentrations in the blood for producing the simultaneous effects of increasing the permeability of the blood-brain barrier and allowing the maximum passage of the exogenous molecule from the blood to the cells of the central nervous system.

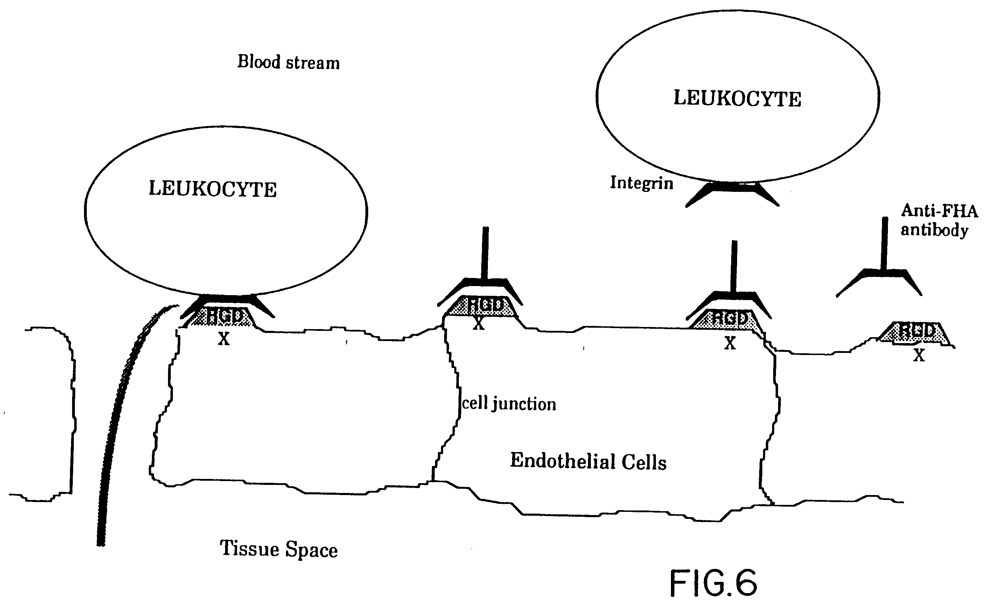

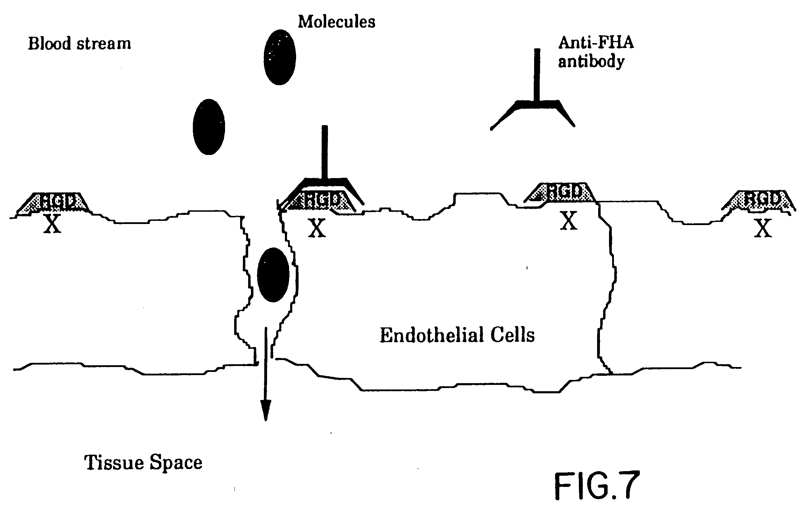

Figs. 6 and 7 illustrate the process by which the antibodies of this invention will enhance permeability of the BBB to water soluble therapeutic agents. The term "therapeutic agents" is used herein as a convenient term to define all of the various materials which a physician or veterinarian will wish to pass through the BBB into the CSF or the brain. It includes, for example anti-infective agents such as antibiotics, antineoplastic agents, diagnostic agents, imaging agents, and immuno-suppressive agents, nerve or glial growth factors and other such products.

Figure 6 shows anti-FHA antibodies in the blood stream adhering to and blocking the RGD region in the x-molecule of endothelial cells. By analogy to the first step in the passage of leukocytes through the endothelia, the blocking of the x-molecule has the effect of opening the cell junctions as shown to the left of the figure. The cell junctions, however, are not open to leukocytes because, as explained above, in order to pass through the opening in the cell wall, a leukocyte must first adhere to the cell by reaction with the x-molecule. It is prevented from so doing because the x-molecule is blocked by the anti-FHA antibody. Figure 7 shows that in the example of Figure 6, other molecules, e.g. the soluble therapeutic agents described above can pass through the open junctions.

Example 1 describes the binding of polyclonal anti-FHA antibodies to the BBB.

Example 2 illustrates this same property for monoclonal antibodies and further teaches that the antibody must bind near the RGD region in a manner like mAb 13.6E2. This property is clearly distinguished from the antibodies 12.5A9 and 12D1 which block the carbohydrate recognition. It also teaches that the antibody 13.6E2 has a preferred affinity for cerebral vessels as opposed to peripheral vessels exemplified by umbilical vein cells.

Example 3 illustrates that the 13.6E2 antibody functions by the mechanism of Figs. 6 and 7 since it blocks entry of leukocytes into CSF at the same time as passage of serum proteins into CSF is enhanced.

Example 4 illustrates that the 13.6E2 antibody enhances blood brain barrier permeability in otherwise healthy animals when injected intravenously.

Examples 5 and 6 show the BBB permeability enhancing properties of 13.6E2 for a therapeutic agent 3H labelled penicillin. The effect is time and dose dependent.

The antibodies of the invention can also serve as carriers for targeting therapeutic agents to endothelial cells in mammals. For this purpose, the therapeutic agent will be chemically bonded to the antibody and the combined product administered to the patient in need of such treatment. These therapeutic agents include, for example coagulation cascade modifiers or immunomodulators such as cytokines. They may include also immunotoxins such as Pseudomonus exotoxin A or ricin attached to an anti-FHA antibody of the invention to produce products capable of killing tumors supplied by or involving vascular endothelium. Procedures for combining such therapeutic agents with proteins such as antibodies are well known.

In certain patients, a potential problem with the use of a murine anti-FHA monoclonal antibody, such as those employed in this invention exists since the patient may generate an immune response against a murine monoclonal antibody. This effect may be ameliorated or obviated by using active fragments of the monoclonal antibody so as to minimize the amount of foreign protein injected. Another alternative is to employ genetic engineering techniques to make a chimeric antibody in which the binding region of the murine anti-FHA antibody is combined with the constant regions of human immunoglobin.

### Compositions

The products of the invention may be provided as parenteral compositions for injection, infusion, or other parenteral procedures, such compositions comprising a prophylactically effective amount of a selected peptide or antibody and a pharmaceutically acceptable carrier. They can, for example be suspended in an inert oil, or in alum or other suitable adjuvant. Alternatively they can be suspended in an aqueous isotonic buffer solution at a pH of about 5.6 to 7.1. Useful buffers include sodium phosphate-phosphoric acid.

The desired isotonicity may be accomplished using sodium chloride or other pharmaceutically acceptable agents such as dextrose, boric acid, sodium tartrate, propylene glycol or other inorganic or organic solutes. Sodium chloride is preferred particularly for buffers containing sodium ions.

If desired the solutions may be thickened with a thickening agent such as methyl cellulose. They may be prepared in emulsified form, either water in oil or oil in water. Any of a wide variety of pharmaceutically acceptable emulsifying agents may be employed including, for example acacia powder, or an alkaryl polyether alcohol sulfate or sulfonate such as a Triton.

The therapeutically useful compositions of the invention are prepared by mixing the ingredients following generally accepted procedures. For example, the selected components may be simply mixed in a blender or other standard device to produce a concentrated mixture which may then be adjusted to the final concentration and viscosity by the addition of water or thickening agent and possibly a buffer to control pH or an additional solute to control tonicity.

The dosage and method of administering the peptides of the invention may be varied due to patient condition, the result sought to be acheived and other factors readily evaluated by the attending physician or veterinarian. For example, while the presently preferred method of administering peptides comprising vaccines of the invention is parenteral administration, certain of them will be administered orally in a pharmaceutically acceptable oral carrier. The antigenic peptides thus administered will generate antibodies in the lymph nodes of the intestine. These antibodies will be distributed systemically to produce a prophylactic effect.

The following non-limiting examples are given by way of illustration only.

### Example 1

### Rabbit and goat polyclonal anti-FHA antibodies bind to cerebral capillary endothelia.

Cerebral capillary endothelial cells harvested from rabbit brains or cryostat sections from human brains were mounted onto glass slides (detailed method appears after the table). Preparations from at least two different individuals were incubated with the antibody at 1:20 dilution (or greater) at room temperature for 2 hrs, rinsed, and incubated for 30 min with either a Vector biotinylated secondary antibody (Vector Elite ABC Immunohistochemistry Kit) for human specimens or a fluresceinated anti-Fc antibody for rabbit specimens. After rinsing, rabbit specimens were viewed with a fluorescence microscope; for human specimens, the Vector avidin-peroxidase mixture was applied for 30 min, followed by rinsing and application of the substrate. The stained tissue preparations were viewed under a light microscope. (+ indicates detectable staining comparable to control).

The results are shown below.

**TABLE I**

| Cross reactivity antibodies to B. pertussis antigens with cerebral capillaries of mammalian brain. | | | |
|---|---|---|---|
| | | Rabbit | Human |
| Goat antisera to: | native purified FHA | + | + |
| | native purified pertussis toxin | 0 | nd |
| Rabbit antisera to: | native purified FHA | + | + |
| | glutaraldehyde-treated FHA | 0 | 0 |
| Human antisera: | pooled cord serum | 0 | 0 |
| | pre-immune (n=8) | 0 | 0 |
| | post-primary DPT (n=2) | + | + |
| | post-infection (n=5) | 3 of 5+ | 3 of 5+ |
| | post-infection absorbed with BP | 0 | 0 |
| | pertussis immune globulin (PIG) | + | + |
| | PIG absorbed with BP | 0 | 0 |
| Controls: | anti-human transferrin receptor | nd | + |
| | rabbit, goat or horse serum | 0 | 0 |

### Details for Table I

Human brain samples were quick frozen 1-3 hrs post mortem; those of animals were frozen at the time of scrifice. For preparation of human and animal tissue slices, cerebral cortex was cut in 20u sections; for some experiments, rabbit cerebral capillaries were extracted from homogenized tissues by centrifugation in 15% dextran. Capillaries or tissue slices were fixed onto glass slides with acetone at room temperature for 10 min and stored at -80°C until used. Antisera were diluted into phosphate buffered saline (PBS) at 1:20. Rabbit capillary staining was visualized with a 1:40 dilution of fluorescenated anti-Fc antibody to the appropriate speices (Boeringher Chemical, Indianapolis, IN). For tissue slices, the Vector ABC Elite immunohistochemistry kit for peroxidase was used to visualize capillary staining. Brain slices were incubated at room temperature in the following: PBS (10 min), 0.3% peroxide/methanol (30 min), PBS (10 min), 0.25% a-methyl mannoside/0.25% nonfat dry milk in PBS (15 min), PBS (10 min). Primary antibody was then applied either at room temperature for 2 hrs orat 4°C for 16 hrs. Slices were rinsed for 10 min in PBS and then treated with the following at room temperature: Vector biotinylated secondary antibody (30 min), PBS (10 min), Vector avidin-peroxidase mixture (30 min), PBS (10 min), peroxidase substrate until color was seen (2-7 min). Purified monoclonal antibodies to human or rat transferrin receptor were always used as a positive control and all observed experimental staining was graded with respect to the positive. Negative controls included goat serum (because the biotinylated anti-rabbit antibodies are made in goat) and horse serum (because the biotinylated anti-rabbit antibodies are made in horse).

### Example 2

### The binding of anti-FHA antibodies to cerebral capillaries involves recognition of the RGD region of FHA.

Monoclonal antibodies were used undiluted as supernatant fluids. Staining of human cerebral capillaries was detected as in Example 1. Each antibody was tested at least two times at 24°C and at 4°C. Staining of capillaries was accompanied by staining of large vessels. The number of + indicates relative degree of staining; antibodies were tested on samples from at least two humans. nd = not determined. Staining of cultured human umbilical vein endothelial cells was performed using peroxidase-anti-peroxidase technique Muller et al, J. Exp. Med. 170:399-404, 1989. The ability of antibodies to bind C3bi was tested in an ELISA assay in which wells were coated with antibodies (10 ug/ml), washed and then incubated for 30 min with erythrocytes coated with C3bi (made as described in Example 8). Captured C3bi-coated erythrocytes were detected visually. The ability of antibodies to block binding of FHA to carbohydrates was performed in an overlay assay as described using glycolipid standards separated by thin layer chromatography [Tuomanen, 1988#260].

The results are shown below.

**TABLE II**

| Ability of monoclonal anti-FHA antibodies to cross react with endothelium, C3bi and inhibit interaction of FHA with carbohydrates in vitro. | | | | |
|---|---|---|---|---|
| | Stain human capillaries | | Bind C3bi | Inhibit FHA-carbohydrate |
| | cerebral | umbilical vein | | |
| Region A | | | | |
| 13.6E2 | +++ | -- | nd | -- |
| 12.5A9 | -- | -- | + | + |
| 12D1 | -- | -- | nd | + |

| Region B | | | | |
|---|---|---|---|---|
| 12.1F9 | -- | -- | +++ | -- |

| Region C | | | | |
|---|---|---|---|---|
| 12.1B11 | -- | + | ++ | -- |

| Region D | | | | |
|---|---|---|---|---|
| 12.6F8 | ++ | + | + | -- |

| Other | | | | |
|---|---|---|---|---|
| G9 | nd | -- | nd | -- |
| A12 | nd | -- | nd | -- |

### Example 3

### Antibody to FHA blocks influx of leukocytes into rabbit cerebrospinal fluid.

Rabbits were inoculated intracisternally with 10⁸ pneumococci and the generation of an inflammatory response in cerebrospinal fluid (CSF) was followed over time by measuring the appearance of leukocytes and protein in CSF. Animals were treated with the antibodies intravenously (2mg/kg of culture supernatant fluid) at the same time as the intracisternal challenge was given.

**TABLE III**

| | leukocyte number* (x 10³/ml CSF) | | | protein** (mg/dl) | |
|---|---|---|---|---|---|
| | 0 | 4 | 5 hrs | 4 | 6 hrs |
| no antibody | 112 | 3661 | 6538 | 1.9 | 2.2 |

| mAb 13.6E2 | | | | | |
|---|---|---|---|---|---|
| (rabbit A) | 162 | 1452+ | 3322+ | 2.9 | 3.3 |
| (rabbit B) | 95 | 1024+ | 2413+ | 1.4 | 3.1 |
| (rabbit C) | 111 | 1098+ | 4435 | 1.4 | 1.8 |

| anti-pertussis toxin (PT) | | | | | |
|---|---|---|---|---|---|
| control Ab | 119 | 3641 | 6245 | 2.3 | 2.8 |

| | | | | | |
|---|---|---|---|---|---|
| *normal is 120; | | | | | |
| **normal is 1.0 | | | | | |
| + significantly different from control | | | | | |

These results are interpreted to show that:
1. Intravenous anti-FHA mAB 13.6E2 significantly decreases the influx of leukocytes into CSF of rabbits challenged with an inflammatory amount of pneumococci. Antibody to pertussis toxin does not. Accumulation of protein in CSF was augmented in antibody treated animals indicating increased blood brain barrier permeability. This latter observation is consistent with Example 4.

### Example 4

### Antibody to FHA enhances permeability of the cerebral capillary endothelium.

Rabbits were injected intravenously with antibodies and the influx of protein into the CSF was followed over time. Such influx occurs only when the permeability of the cerebral capillary endothelium is enhanced. As shown in Example 3, such protein influx was enhanced during the inflammatory response to bacterial products in CSF in antibody treated animals. In this example, this activity was demonstrated in the absence of an inflammatory stimulus.

**TABLE IV**

| | | protein (mg/dl) | |
|---|---|---|---|
| | | 4 | 6 hrs |
| no antibody | | 0.8 | 0.8 |
| mAb 13.6E2 | (rabbit A) | 2.2 | 3.7 |
| | (rabbit B) | 2.0 | 1.8 |
| | (rabbit C) | 1.4 | 1.8 |
| polyclonal anti FHA Ab | | 1.2 | 1.2 |

These results are interpreted to show that:
Anti-FHA antibodies, particularly the monoclonal antibody 13.6E2, enhance the permeability of cerebral capillary endothelium sufficiently as to allow passage of serum proteins into CSF.

### Example 5

### Comparative Activity Of Anti-FHA Antibodies In Enhancing Penetration Of [³H] Penicillin From Serum To Brain

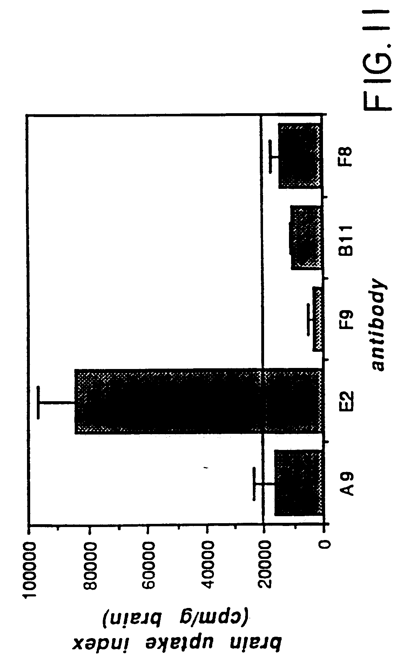

Rabbits (n= at least 2 per condition) were injected with the selected antibodies identified in Example 2 (10 ug/kg) intravenously; 4 hours later, ³Hpenicillin (1.04 mCu/50ug/rabbit; Merck, Sharp and Dohme, Rahway, NJ) was injected intravenously and after 30 min, blood and brain harvested. Approximately one gram of right parietal cortex was excised, weighed, homogenized, solubilized with Soluene 350 (Packard, Boston, MA), and ³H cpm in brain and serum samples were counted. Blood volume per gram of brain was determined to be 300± 73ul from a set of 6 saline-treated control animals. Brain uptake index indicates the amount of radiolabel in brain tissue after subtraction of values from blood (BUI = cpm per gram brain homogenate - [cpm per ul blood x 300 ul blood/gram homogenate]). Results are shown in Fig. 11. The horizontal line indicates the maximum uptake observed in the IV saline control group (n=6). The ³H penicillin is ±80% bound to albumin and thus, accumulation of radiolabel in this model reflects permeability of the BBB to large soluble molecules the size of albumin (70kD). Only anti-FHA antibody 13.6E2 increased the passage of penicillin into brain.

### Example 6

### Antibody 13.6E2 Enhances The Permeability Of The Cerebral Capillary Endothelium In A Reversible, Time Dependent Manner

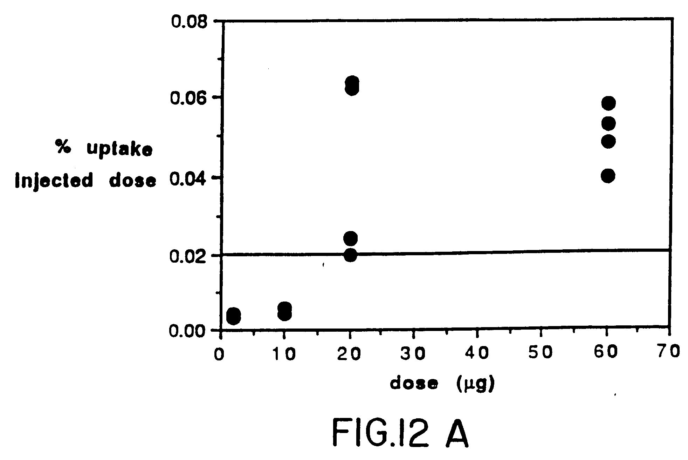

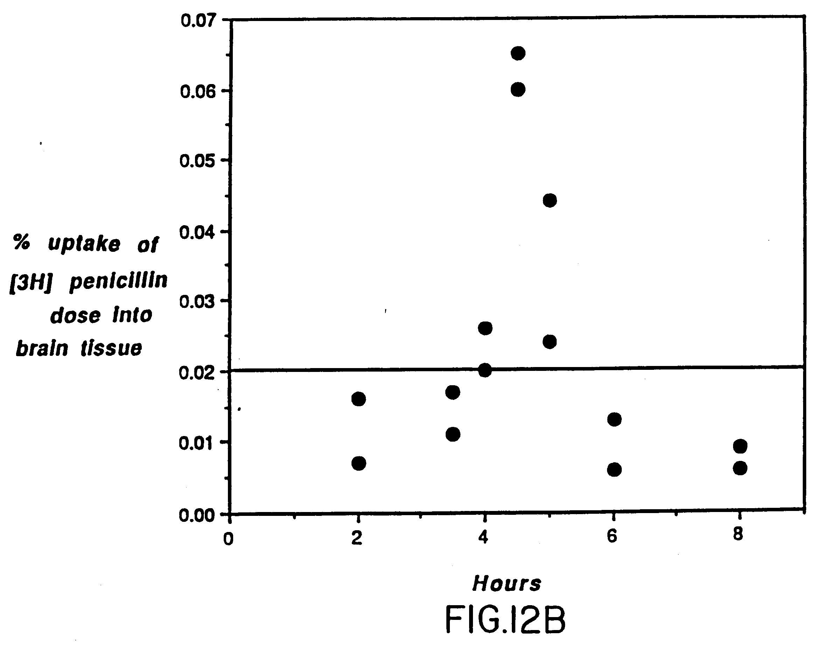

Healthy Animals received antibody 13.6E2 intravenously (10ug/kg) followed at various times thereafter by ³H penicillin intravenously. Thirty minutes later, blood and brain were harvested and analyzed as in Example 5. Results from each animal are plotted individually in Figure 12; cpm/g brain was converted to percent of the initial injected dose of radiolabel/entire brain weight.

### Example 7

| Intravenous Formulation I | |
|---|---|
| Ingredient | mg/ml |
| cefotaxime | 250.0 |
| monoclonal antibody 12.5D1 | 10.0 |
| dextrose USP | 45.0 |
| sodium bisulfite USP | 3.2 |
| edetate disodium USP | 0.10 |
| water for injection q.s.a.d. | 1.00 ml |

| Intravenous Formulation II | |
|---|---|
| Ingredient | mg/ml |
| ampicillin | 250.0 |
| monoclonal antibody 12.1F9 | 10.0 |
| sodium bisulfite USP | 3.2 |
| disodium edetate USP | 0.1 |
| water for injection q.s.a.d. | 1.00 ml |

| Intravenous Formulation III | |
|---|---|
| Ingredient | mg/ml |
| gentamicin (charged as sulfate) | 40.0 |
| monoclonal antibody 12.1 B11 | 10.0 |
| sodium bisulfite USP | 3.2 |
| disodium edetate USP | 0.1 |
| water for injection q.s.a.d. | 1.00 ml |

### Example 8

### C3bi Capture Test

The presence of anti-C3bi antibodies in a serum to FHA is tested by binding the antibodies in the serum to a surface of a plate coated with Protein A and adding C3bi coated particles. The addition of C3bi coated particles leads to capture of the particles if the serum contains anti-C3bi antibodies.
The test is performed in the following manner:

### Preparation of C3bi coated erythrocytes (EC3bi)

1. Put E^{IgM} (amount see below) in a 15 ml tube
spin 5 min 2000 rpm
aspirate supernatant
resuspend in DGVB++ (DG Veronal Buffer with divalent cations; amount see below)
add C5 deficient serum

| | Amount of E^{IgM} | Amount of DGVB++ | Amount of C5 deficient serum |
|---|---|---|---|
| Super | 250 ul | 250 ul | 40 ul |
| High | 250 ul | 250 ul | 25 ul |
| Medium | 500 ul | 250 ul | 25 ul |
| Low | 250 ul | 80 ul | 8 ul |

2. Incubate 60 min 37°, shake the tubes after 30 min.
3. Stop with 500 ul EDTA/GVB--(G Veronal Buffer without divalent cations)
4. Incubate 10 min 0°C (on ice)
5. Wash 4x in DGVB++
therefore add 5 ml DVGB++, spin 5 min, 2000 rpm,
4°C aspirate supernatant
6. Resuspend in:
Super 2.5 ml DGVB++
High 2.5 ml DGVB++
Medium 5.0 ml DGVB++
Low 2.5 ml DGVB++
Concentration is now 1x10⁸/ml
7. Finally, add for storage to E^{IgM} and EC3bi, 1ul penicillin/streptomicin per 100 ul of suspension. Store ice. Suspensions are usable for 3 weeks.

### IgG capture on Protein A and Toxicity test

1. Coat each well of a 60 well high profile terasaki tray 1003-01-0) with 5 ul of commercial protein A solution (conc 50 ug/ml) for 2 hr. at RT.
2. Adjust the pH of serum/fluid to 8.0 by adding 1/10 volume of 1.0M Tris pH 8.0.
3. Wash the wells 2 times with Tris (1M) pH 8.0, aspirate all fluid.
4. Add the undiluted test serum (5ul) and leave for 2 hr.
5. Wash 2 times with PBS pH 7.4.
6. Remove fluid by aspiration
7. Add 5 ul C3bi supra coated erythrocytes diluted in PBS as described above.
8. Incubate for 30 min at 37°C in the incubator.
9. After 30 min incubation, turn the plates upside down to allow gravity to remove unbound erythrocytes for 10 min at 37°C.
10. Wash with PBS 3 times, slam on paper, wash once more.
11. Remove the fluid by aspiration
12. Add glutaraldehyde (2.5%) in PBS for 2 minutes.
13. Wash with PBS 3 times
14. Count at magnification 400 (100 fields).

PBS=with Ca and Mg.
PBS/glutaraldehyde = 1 ml 25% glutaraldehyde into 9 ml PBS.
Binding at or above the level of the positive control commercially available anti-C3bi or mAbF9 indicates toxic antibodies are present.

### Ability of anti-B, pertussis antisera to bind cerebral capillaries and C3bi

| Antigen | Species | Brain Cap* | C3bi Capture |
|---|---|---|---|
| preimmune | guinea pig | -- | -- |
| | rabbit | | -- |
| | human | -- | -- |
| DPT (whole cell) | guinea pig | +++ | + |
| | rabbit | | ++ |
| | human | +++ | + |
| PT (MAPT) (JNIH-7) | guinea pig | ++ | -- |
| | human | ++ | -- |
| Native FHA | guinea pig | + | + |
| | rabbit | | + |
| | human | | |
| Formalin FHA | guinea pig | + | + |
| | rabbit | | -- |
| | human | | |
| JNIH-6 | guinea pig | (--) | (--) |
| | rabbit | | |
| | human | + | + |
| FHA, TNM | guinea pig | ++ | -- |
| | rabbit | | |
| | human | | |

| | | | |
|---|---|---|---|
| *Binding to cerebral capillaries was performed as described in Example 1. | | | |
| DPT Diphteria Pertussis Tetanus PT Pertussis Toxin TNM Trinitromethane | | | |

The application of this test to anti-FHA monoclonal antibodies is shown in Fig. 10.

## Claims

1. A peptide selected from those that contain or mimic the arginyl-glycyl-aspartyl (RGD) region, Factor Ten region or the C3bi region of filamentous hemagglutinin (FHA) and will inhibit adhesion between leukocytes and endothelia, wherein the peptide is selected from: 
IGALKAGAVEAASPRRARRALRQDFFTPGSVVVRAQGNVTVGRGDP,
RQDFFTPGSVVVRAQGNVTVGRGDPMQGVLAQGDIIMDAKGGTLL,
RGDPMQGVLAQGDIIMDAKGGTLLLRNDALTEMGTVTISADSAVL,
GRTRG,
ETKEVDG,
GLIQGRSVKVD,
LGYQAK,
 and analogs thereof.

2. An antibody to a peptide or analog thereof according to claim 1, in particular a polyclonal or monoclonal antibody.

3. A pharmaceutical composition comprising a peptide or analog thereof selected from those that contain or mimic the RGD region, Factor Ten region or the C3bi region of FHA and will inhibit adhesion between leukocytes and endothelia, provided said peptide or analog thereof is not full length FHA, and a pharmaceutically acceptable carrier.

4. Composition according to claim 3, wherein the peptide is selected from: 
IGALKAGAVEAASPRRARRALRQDFFTPGSVVVRAQGNVTVGRGDP,
RQDFFTPGSVVVRAQGNVTVGRGDPMQGVLAQGDIIMDAKGGTLL,
RGDPMQGVLAQGDIIMDAKGGTLLLRNDALTEMGTVTISADSAVL,
GRTRG,
ETKEVDG,
GLIQGRSVKVD,
LGYQAK,
 peptides which mimic said peptides and analogs thereof.

5. Composition according to any one of claims 3 or 4, further comprising a therapeutically effective amount of another therapeutic agent.

6. Composition according to claim 5, wherein said therapeutic agent is chemically bonded to said peptide.

7. A pharmaceutical composition comprising an antibody to FHA or to a peptide or analog thereof selected from those that contain or mimic the RGD region, Factor Ten region or the C3bi region of FHA and will inhibit adhesion between leukocytes and endothelia, and a pharmaceutically acceptable carrier.

8. Composition according to claim 7 suitable for increasing the permeability of the blood-brain barrier to a molecule present in the bloodstream of the host, wherein the antibody is an antibody to FHA or to a peptide or analog thereof which contains or mimics the RGD region of FHA.

9. Composition according to claim 8, further comprising the molecule to be delivered from the blood to the brain.

10. Composition according to any one of claims 8 or 9, wherein the molecule comprises an optionally radiolabeled diagnostic imaging agent or a neuropharmaceutical agent.

11. Composition according to any one of claims 8 to 10, wherein the antibody binds to the x-molecule on brain endothelium.

12. Composition according to any one of claims 8 to 11, wherein the antibody is a monoclonal antibody.

13. Composition according to any one of claims 8 to 12, further comprising a therapeutically effective amount of another therapeutic agent.

14. Composition according to any one of claims 3 to 13, wherein the pharmaceutically acceptable carrier is a carrier for intravenous administration.

15. Use of FHA, a peptide of FHA or analog thereof selected from those that contain or mimic the RGD region, Factor Ten region or the C3bi region of FHA, for the preparation of a pharmaceutical for inhibiting the influx of leukocytes into inflamed tissue and/or for targeting therapeutic agents to leucocytes.

16. Use according to claim 15, wherein said FHA, peptide of FHA or analog thereof is capable of inhibiting adhesion between a leukocyte and the RGD region, the Factor Ten region or the C3bi region of FHA.

17. Use according to any one of claims 15 or 16, wherein the peptide is selected from: 
IGALKAGAVEAASPRRARRALRQDFFTPGSVVVRAQGNVTVGRGDP,
RQDFFTPGSVVVRAQGNVTVGRGDPMQGVLAQGDIIMDAKGGTLL,
RGDPMQGVLAQGDIIMDAKGGTLLLRNDALTEMGTVTISADSAVL,
GRTRG,
ETKEVDG,
GLIQGRSVKVD,
LGYQAK,
 peptides which mimic said peptides and analogs thereof.

18. Use of an antibody to FHA or to a peptide or analog thereof selected from those that contain or mimic the RGD region, Factor Ten region or the C3bi region of the FHA, that will inhibit adhesion between leukocytes and endothelia, for the preparation of a pharmaceutical for inhibiting the influx of leukocytes into inflamed tissue and/or for increasing the permeability of the blood-brain barrier to a molecule present in the bloodstream of the host.

19. Use according to claim 18, wherein the antibody is a blood-brain barrier permeabilizing antibody to FHA or a peptide or analog thereof which contains or mimics the RGD region of FHA.

20. Use according to claim 19, wherein the antibody is bindable to the x-molecule in brain endothelium.

21. Use according to any one of claims 18 to 20, wherein the host is a human being.

22. Use according to any one of claims 15 to 21, wherein the pharmaceutical is for parenteral administration.

## Patentansprüche

1. Peptid, ausgewählt aus solchen, die die Arginyl-Glycyl-Aspartyl(RGD)-Region, die Faktor X-Region oder die C3bi-Region von filamentösem Hämagglutinin (FHA) enthalten oder nachbilden und die Adhäsion zwischen Leukozyten und Endothel inhibieren, wobei das Peptid ausgewählt ist aus: 
IGALKAGAVEAASPRRARRALRQDFFTPGSVVVRAQGNVTVGRGDP,
RQDFFTPGSVVVRAQGNVTVGRGDPMQGVLAQGDIIMDAKGGTLL,
RGDPMQGVLAQGDIIMDAKGGTLLLRNDALTEMGTVTISADSAVL,
GRTRG,
ETKEVDG,
GLIQGRSVKVD,
LGYQAK,
 und Analoga davon.

2. Antikörper gegen ein Peptid oder dessen Analogon nach Anspruch 1, insbesondere ein polyklonaler oder monoklonaler Antikörper.

3. Pharmazeutische Zusammensetzung, umfassend ein Peptid oder Analogon davon, ausgewählt aus solchen, die die RGD-Region, die Faktor X-Region oder die C3bi-Region von FHA enthalten oder nachbilden und die Adhäsion zwischen Leukozyten und Endothel inhibieren, mit der Einschränkung, daß es sich bei dem Peptid oder dessen Analogon nicht um FHA mit voller Länge handelt, sowie einen pharmazeutisch verträglichen Trägerstoff.

4. Zusammensetzung nach Anspruch 3, worin das Peptid ausgewählt ist aus: 
IGALKAGAVEAASPRRARRALRQDFFTPGSVVVRAQGNVTVGRGDP,
RQDFFTPGSVVVRAQGNVTVGRGDPMQGVLAQGDIIMDAKGGTLL,
RGDPMQGVLAQGDIIMDAKGGTLLLRNDALTEMGTVTISADSAVL,
GRTRG,
ETKEVDG,
GLIQGRSVKVD,
LGYQAK,
 Peptiden, die diese Peptide nachbilden und Analoga davon.

5. Zusammensetzung nach irgendeinem der Ansprüche 3 oder 4, zusätzlich umfassend eine therapeutisch wirksame Menge eines weiteren Arzneimittels.

6. Zusammensetzung nach Anspruch 5, worin das Arzneimittel chemisch an das Peptid gebunden ist.

7. Pharmazeutische Zusammensetzung, umfassend einen Antikörper gegen FHA oder ein Peptid oder Analogon davon, ausgewählt aus solchen, die die RGD-Region, die Faktor X-Region oder die C3bi-Region von FHA enthalten oder nachbilden und die Adhäsion zwischen Leukozyten und Endothel inhibieren, sowie einen pharmazeutisch verträglichen Trägerstoff.

8. Zusammensetzung nach Anspruch 7, die geeignet ist, die Permeabilität der Blut-Hirn-Schranke für ein im Blut des Wirts vorhandenes Molekül zu erhöhen, wobei der Antikörper ein Antikörper gegen FHA oder gegen ein Peptid oder Analogon davon ist, das die RGD-Region von FHA enthält oder nachbildet.

9. Zusammensetzung nach Anspruch 8, zusätzlich umfassend das Molekül, das vom Blut zum Hirn transportiert werden soll.

10. Zusammensetzung nach irgendeinem der Ansprüche 8 oder 9, worin das Molekül ein gegebenenfalls radiomarkiertes Mittel für diagnostische bildgebende Verfahren oder ein Neuropharmazeutikum umfaßt.

11. Zusammensetzung nach irgendeinem der Ansprüche 8 bis 10, worin der Antikörper an das x-Molekül an Hirn-Endothel bindet.

12. Zusammensetzung nach irgendeinem der Ansprüche 8 bis 11, worin der Antikörper ein monoklonaler Antikörper ist.

13. Zusammensetzung nach irgendeinem der Ansprüche 8 bis 12, zusätzlich umfassend eine therapeutisch wirksame Menge eines weiteren Arzneimittels.

14. Zusammensetzung nach irgendeinem der Ansprüche 3 bis 13, worin der pharmazeutisch verträgliche Trägerstoff ein Trägerstoff zur intravenösen Verabreichung ist.

15. Verwendung von FHA, einem FHA-Peptid oder einem Analogon davon, ausgewählt aus solchen, die die RGD-Region, die Faktor X-Region oder die C3bi-Region von FHA enthalten oder nachbilden, zur Herstellung eines Medikaments zur Inhibierung der Leukozyteneinwanderung in entzündetes Gewebe und/oder um Arzneimittel zu Leukozyten zu dirigieren.

16. Verwendung nach Anspruch 15, worin das FHA, FHA-Peptid oder Analogon davon befähigt ist, die Adhäsion zwischen einem Leukozyten und der RGD-Region, der Faktor X-Region oder der C3bi-Region von FHA zu inhibieren.

17. Verwendung nach irgendeinem der Ansprüche 15 oder 16, worin das Peptid ausgewählt ist aus: 
IGALKAGAVEAASPRRARRALRQDFFTPGSVVVRAQGNVTVGRGDP,
RQDFFTPGSVVVRAQGNVTVGRGDPMQGVLAQGDIIMDAKGGTLL,
RGDPMQGVLAQGDIIMDAKGGTLLLRNDALTEMGTVTISADSAVL,
GRTRG,
ETKEVDG,
GLIQGRSVKVD,
LGYQAK,
 Peptiden, die diese Peptide nachbilden und Analoga davon.

18. Verwendung eines Antikörpers gegen FHA oder gegen ein Peptid oder Analogon davon, ausgewählt aus solchen, die die RGD-Region, die Faktor X-Region oder die C3bi-Region von FHA enthalten oder nachbilden, der die Adhäsion zwischen Leukozyten und Endothel inhibiert, zur Herstellung eines Medikaments zur Inhibierung der Leukozyteneinwanderung in entzündetes Gewebe und/oder zur Erhöhung der Permeabilität der Blut-Hirn-Schranke für ein im Blut des Wirts vorhandenes Molekül.

19. Verwendung nach Anspruch 18, worin der Antikörper ein die Blut-Hirn-Schranke permeabilisierender Antikörper gegen FHA oder ein Peptid oder Analogon davon ist, das die RGD-Region von FHA enthält oder nachbildet.

20. Verwendung nach Anspruch 19, worin der Antikörper an das x-Molekül im Hirn-Endothel binden kann.

21. Verwendung nach irgendeinem der Ansprüche 18 bis 20, wobei es sich bei dem Wirt um einen Menschen handelt.

22. Verwendung nach irgendeinem der Ansprüche 15 bis 21, worin das Medikament zur parenteralen Verabreichung ist.

## Revendications

1. Un peptide sélectionné de ceux qui contiennent ou miment la région arginyle-glycyle-aspartyle (RGD), la région Facteur Dix ou la région C3bi d'hémagglutinine filamenteuse (FHA) et empêchent l'adhésion entre des leucocytes et des endothélia, dans lequel le peptide est sélectionné de: 
IGALKAGAVEAASPRRARRALRQDFFTPGSVVVRAQGNVTVGRGDP,
RQDFFTPGSVVVRAQGNVTVGRGDPMQGVLAQGDIIMDAKGGTLL,
RGDPMQGVLAQGDIIMDAKGGTLLLRNDALTEMGTVTISADSAVL,
GRTRG,
ETKEVDG,
GLIQGRSVKVD,
LGYQAK,
 et des analogues de celui-ci.

2. Un anticorps à un peptide ou un analogue de celui-ci selon la revendication 1, en particulier un anticorps polyclonal ou monoclonal.

3. Une composition pharmaceutique comprenant un peptide ou un analogue de celui-ci sélectionné de ceux qui contiennent ou miment la région RGD, la région Facteur Dix ou la région C3bi de FHA et empêchent l'adhésion entre des leucocytes et des endothélia, pourvu que ledit peptide ou un analogue de celui-ci n'ait pas l'entière longueur FHA, et un porteur pharmaceutiquement acceptable.

4. Une composition selon la revendication 3, dans laquelle le peptide est sélectionné de: 
IGALKAGAVEAASPRRARRALRQDFFTPGSVVVRAQGNVTVGRGDP,
RQDFFTPGSVVVRAQGNVTVGRGDPMQGVLAQGDIIMDAKGGTLL,
RGDPMQGVLAQGDIIMDAKGGTLLLRNDALTEMGTVTISADSAVL,
GRTRG,
ETKEVDG,
GLIQGRSVKVD,
LGYQAK,
 des peptides qui miment lesdits peptides et des analogues de ceux-ci.

5. Une composition selon l'une quelconque des revendications 3 ou 4, comprenant de plus un montant thérapeutiquement effectif d'un autre agent thérapeutique.

6. Une composition selon la revendication 5, dans laquelle ledit agent thérapeutique et chimiquement lié audit peptide.

7. Une composition pharmaceutique comprenant un anticorps à FHA ou à un peptide ou un analogue de celui-ci sélectionné de ceux qui contiennent ou miment la région RGD, la région Facteur Dix ou la région C3bi de FHA et empêchent l'adhésion entre des leucocytes et des endothélia, et un porteur pharmaceutiquement acceptable.

8. Une composition selon la revendication 7, susceptible d'augmenter la perméabilité de la barrière sang-cerveau à une molécule présente dans la circulation sanguine de l'hôte, dans laquelle l'anticorps est un anticorps à FHA ou à un peptide ou un analogue de celui-ci qui contient ou mime la région RGD de FHA.

9. Une composition selon la revendication 8, comprenant de plus la molécule à être délivrée du sang au cerveau.

10. Une composition selon l'une quelconque des revendications 8 ou 9, dans laquelle la molécule comprend un agent d'image diagnostique radiomarqué faculativement ou un agent neuropharmaceutique.

11. Une composition selon l'une quelconque des revendications 8 à 10, dans laquelle l'anticorps se lie à la molécule x sur l'endothélium du cerveau.

12. Une composition selon l'une quelconque des revendications 8 à 11, dans laquelle l'anticorps est un anticorps monoclonal.

13. Une composition selon l'une quelconque des revendications 8 à 12, comprenant de plus un montant thérapeutiquement effectif d'un autre agent thérapeutique.

14. Une composition selon l'une quelconque des revendications 8 à 13, dans laquelle le porteur pharmaceutiquement acceptable et un porteur pour l'administration intraveineuse.

15. Une utilisation de FHA, un peptide de FHA ou un analogue de celui-ci sélectionné de ceux qui contiennent ou miment la région RGD, la région Facteur Dix ou la région C3bi de FHA, pour la préparation d'un pharmaceutique pour empêcher l'entrée de leucocytes dans un tissu enflammé et/ou l'acheminement d'agents thérapeutiques aux leucocytes.

16. Une utilisation selon la revendication 15, dans laquelle ledit FHA, peptide de FHA ou un analogue de celui-ci est capable d'empêcher l'adhésion entre un leucocyte et la région RGD, la région Facteur Dix ou la région C3bi de FHA.

17. Une utilisation selon l'une quelconque des revendications 15 ou 16, dans laquelle le peptide est sélectionné de: 
IGALKAGAVEAASPRRARRALRQDFFTPGSVVVRAQGNVTVGRGDP,
RQDFFTPGSVVVRAQGNVTVGRGDPMQGVLAQGDIIMDAKGGTLL,
RGDPMQGVLAQGDIIMDAKGGTLLLRNDALTEMGTVTISADSAVL,
GRTRG,
ETKEVDG,
GLIQGRSVKVD,
LGYQAK,
 des peptides qui miment lesdits peptides ou des analogues de ceux-ci.

18. Une utilisation d'un anticorps à FHA ou un peptide ou un analogue de celui-ci sélectionné de ceux qui contiennent ou miment la région RGD, la région Facteur Dix ou la région C3bi de FHA, qui empêche l'adhésion entre des leucocytes et des endothélia, pour la préparation d'un pharmaceutique pour empêcher l'entrée de leucocytes dans un tissu enflammé et/ou pour augmenter la perméabilité de la barrière sang-cerveau à une molécule présente dans la circulation sanguine de l'hôte.

19. Une utilisation selon la revendication 18, dans laquelle l'anticorps est un anticorps rendant perméable la barrière sang-cerveau, à FHA ou un peptide ou un analogue de celui-ci qui contient ou mime la région RGD de FHA.

20. Une utilisation selon la revendication 19, dans laquelle l'anticorps peut être lié à la molécule x dans l'endothélium du cerveau.

21. Une utilisation selon l'une quelconque des revendications 18 à 20, dans laquelle l'hôte est un être humain.

22. Une utilisation selon l'une quelconque des revendications 15 à 21, dans laquelle le pharmaceutique est pour l'administration parentérale.
